(19) Europäisches Patentamt
European Patent Office
Office européen des brevets

(11) Veröffentlichungsnummer: **0 415 185 B1**

(12) **EUROPÄISCHE PATENTSCHRIFT**

(45) Veröffentlichungstag der Patentschrift: **07.12.94**

(51) Int. Cl.$^5$: **C07D 207/38**, A01N 43/36

(21) Anmeldenummer: **90115656.2**

(22) Anmeldetag: **16.08.90**

# (54) 4-Alkoxy- bzw. 4-(substituierte)Amino-3-arylpyrrolinon-Derivate.

(30) Priorität: **29.08.89 DE 3928504**

(43) Veröffentlichungstag der Anmeldung:
**06.03.91 Patentblatt 91/10**

(45) Bekanntmachung des Hinweises auf die Patenterteilung:
**07.12.94 Patentblatt 94/49**

(84) Benannte Vertragsstaaten:
**BE CH DE FR GB IT LI NL**

(56) Entgegenhaltungen:
**EP-A- 0 262 399**

**LIEBIGS ANN. CHEM., 1985, Seiten 1095-1098, VCH Verlagsgesellschaft mbH,Weinheim, DE; R. SCHMIERER et al.: "Cyclisierung von N-Acylalanin- und N-Acylglycinestern"**

(73) Patentinhaber: **BAYER AG**

**D-51368 Leverkusen (DE)**

(72) Erfinder: **Baasner, Bernd, Dr.**
**Wagner-Strasse 83**
**D-5060 Bergisch Gladbach 2 (DE)**
Erfinder: **Fischer, Reiner, Dr.**
**Nelly-Sachs-Strasse 23**
**D-4019 Monheim (DE)**
Erfinder: **Lürssen, Klaus, Dr.**
**August-Kierspel-Strasse 145**
**D-5060 Bergisch Gladbach 2 (DE)**
Erfinder: **Santel, Hans-Joachim, Dr.**
**Grünstrasse 9a**
**D-5090 Leverkusen 1 (DE)**
Erfinder: **Schmidt, Robert R., Dr.**
**Im Waldwinkel 110**
**D-5060 Bergisch Gladbach 2 (DE)**

**EP 0 415 185 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 4-Alkoxy- bzw. 4-(substituierte)Amino-3-arylpyrrolinon-Derivate, Verfahren sowie neue Zwischenprodukte zu ihrer Herstellung und ihre Verwendung als Herbizide.

Von 3-Acyl-pyrrolidin-2,4-dionen sind pharmazeutische Eigenschaften vorbeschrieben (S. Suzuki et. al., Chem. Pharm. Bull. 15 1120 (1967)). Weiterhin wurden N-Phenylpyrrolidin-2,4-dione von R. Schmierer und H. Mildenberger, Liebigs Ann. Chem. 1985 1095, synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide Wirksamkeit bekannt geworden ist.

Weiterhin werden in der zu der vorliegenden Anmeldung nicht vorveröffentlichen deutschen Patentanmeldung P 3 900 301.9 vom 7. Januar 1989 3-Arylpyrrolidin-2,4-dion-Derivate mit insektizider, akarizider und herbizider Wirkung beschrieben, in denen in 4-Stellung des Pyrrolinons Hydroxy stehen kann. In der o.g. Anmeldung DE-P 3 900 301.9 wörtlich genannte Verbindungen werden mittels Disclaimers vom Schutzumfang der vorliegenden Anmeldung ausgeschlossen.

Es wurden nun neue 4-Alkoxy- bzw. 4-(substituierte) Amino-3-arylpyrrolinon-Derivate der allgemeinen Formel (I) gefunden

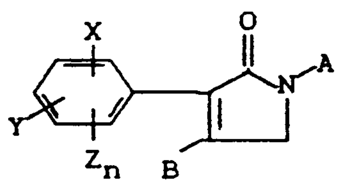

(I)

in welcher

X und Y unabhängig voneinander für Wasserstoff, Fluor, Chlor, Brom, Iod, $C_1$-$C_6$-Alkyl, $C_1$-$C_6$-Alkoxy, Halogen-$C_1$-$C_4$-alkyl, jeweils unsubstituiertes oder einfach bis fünffach, gleich oder verschieden substituiertes Phenoxy oder Phenylthio stehen, wobei als Phenylsubstituenten ausgewählt sind: Fluor, Chlor, Brom, Iod, $C_1$-$C_4$-Alkyl, $C_1$-$C_4$-Alkoxy und Halogen-$C_1$-$C_4$-alkyl,

Z für Fluor, Chlor, Brom, Iod, $C_1$-$C_6$-Alkyl oder $C_1$-$C_6$-Alkoxy steht,

n für eine Zahl 0, 1, 2 oder 3 steht,

A für jeweils unsubstituiertes oder jeweils durch Halogen substituiertes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_1$-$C_8$-alkyl, $C_1$-$C_{10}$-Alkylthio-$C_1$-$C_8$-alkyl oder für Cycloalkyl mit 3 bis 8 Ringatomen steht, das durch Sauerstoff, Stickstoff und/oder Schwefel unterbrochen sein kann, oder A für jeweils unsubstituiertes oder im Phenylteil einfach bis fünffach, gleich oder verschieden substituiertes Phenyl-$C_1$-$C_6$-alkyl, Phenyl oder Naphthyl steht, wobei als Substituenten jeweils ausgewählt sind: Fluor, Chlor, Brom, Iod, $C_1$-$C_6$-Alkyl, $C_1$-$C_6$-Alkoxy, Halogen-$C_1$-$C_6$-alkyl, Halogen-$C_1$-$C_6$-alkoxy oder jeweils unsubstituiertes oder einfach bis fünffach, gleich oder verschieden durch Fluor, Chlor, Brom, Iod, $C_1$-$C_4$-Alkyl, $C_1$-$C_4$-Alkoxy, Halogen-$C_1$-$C_4$-alkyl und Halogen-$C_1$-$C_4$-alkoxy substituiertes Phenoxy oder Phenylthio,

B für Hydroxy, $C_1$-$C_{10}$-Alkoxy, Halogen-$C_1$-$C_{10}$-alkoxy oder Cycloalkoxy mit 3 bis 7 Ringatomen steht, wobei der Cycloalkyl-Rest unsubstituiert oder einfach bis fünffach, gleich oder verschieden durch Fluor, Chlor, Brom und $C_1$-$C_4$-Alkyl substituiert ist, oder

B für die Gruppe

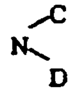

steht, wobei

C und D unabhängig voneinander für Wasserstoff, $C_1$-$C_{10}$-Alkyl, $C_3$-$C_{10}$-Alkenyl, $C_1$-$C_8$-Alkoxy-$C_1$-$C_8$-alkyl, $C_1$-$C_8$-Alkylthio-$C_1$-$C_8$-alkyl, $C_1$-$C_8$-Polyalkoxy-$C_1$-$C_8$-alkyl, Cycloalkyl mit 3 bis 8 Ringatomen, das durch ein, zwei oder drei gleiche oder verschiedene Sauerstoff-, Stickstoff-

und Schwefelatome unterbrochen sein kann, jeweils unsubstituiertes oder einfach bis fünffach, gleich oder verschieden substituiertes Phenyl, Phenyl-$C_1$-$C_6$-alkyl, Phenoxy-$C_1$-$C_6$-alkyl, Hetaryl oder Hetaryl-$C_1$-$C_6$-alkyl mit jeweils 5 oder 6 Ringatomen, wobei 1 oder 2 gleiche oder verschiedene Sauerstoff-, Stickstoff- und Schwefelatome im Ring enthalten sind, oder

C und D zusammen mit dem Stickstoff, an den sie gebunden sind, einen 3- bis 8-gliedrigen Ring bilden, der ein oder zwei Doppelbindungen enthalten kann, der ein oder zwei weitere, gleiche oder verschiedene Sauerstoff-, Schwefel- oder Stickstoffatome enthalten kann, und der einfach bis fünffach, gleich oder verschieden durch $C_1$-$C_4$-Alkyl und $C_1$-$C_4$-Alkylcarbonyl substituiert sein kann,

mit der Maßgabe, daß wenigstens ein Rest X, Y oder Z nicht für Chlor oder Methyl steht, wenn B für Hydroxy steht und A eine andere Bedeutung als gegebenenfalls substituiertes Phenyl oder Naphthyl hat und wenigstens ein Rest X, Y oder Z eine andere Bedeutung als Wasserstoff hat, wenn B für Hydroxy steht und A die Bedeutung von 2,6-Diethylphenyl hat.

Die aliphatischen Kohlenstoffketten, wie beispielsweise Alkyl, Halogenalkyl, Arylalkyl oder Phenoxyalkyl sind jeweils geradkettig oder verzweigt.

Bei substituierten Systemen, wie beispielsweise substituiertes Alkyl, Alkenyl, Cycloalkyl oder Aryl, kann die Substitution jeweils einfach oder mehrfach, gleich oder verschieden erfolgen. Aromatische Systeme werden bevorzugt einfach bis fünffach, insbesondere einfach bis dreifach, cyclische Systeme bevorzugt einfach bis achtfach, insbesondere einfach bis fünffach, substituiert.

Weiterhin wurde gefunden, daß man die Verbindungen der Formel (I), in denen

a) B für Hydroxyl steht und in welcher die Reste A, X, Y und $Z_n$ die oben angegebene Bedeutung haben, erhält, wenn man

N-Acylaminosäureester der Formel (II)

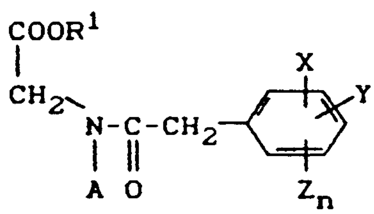

(II)

in welcher

A, X, Y, Z und n die oben angegebene Bedeutung haben, und

$R^1$ für Alkyl, insbesondere Methyl oder Ethyl, steht,

in Gegenwart eines Verdünnungsmittels und in Gegenwart einer Base intramolekular kondensiert,

oder daß man

b) Verbindungen der Formel (I), in denen

B für Alkoxy, Halogenalkoxy oder Cycloalkoxy steht, wobei der Cycloalkyl-Rest unsubstituiert oder durch Halogen und/oder Alkyl substituiert ist, und

A, X, Y, Z und n die oben angegebene Bedeutung haben,

erhält, wenn man Verbindungen der Formel (I), in denen B für Hydroxyl steht,

mit Alkylierungsreagenzien der Formel (III)

$R^2$ - $X^1$ (III)

in welcher

$R^2$ für Alkyl, Halogenalkyl oder Cycloalkyl steht, wobei der Cycloalkyl-Rest unsubstituiert oder durch Halogen und/oder Alkyl substituiert ist, und

$X^1$ für Halogen oder eine andere für Alkylierungsreagenzien übliche Abgangsgruppe steht,

gegebenenfalls in Gegenwart eines Verdünnungsmittels und gegebenenfalls in Gegenwart eines Säurebindemittels umsetzt,

oder daß man

c) Verbindungen der Formel (I), in denen
B für

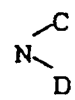

steht und
A, C, D, X, Y, Z und n die oben angegebene Bedeutung haben,
erhält, wenn man Verbindungen der Formel (I), in denen B für Hydroxyl steht,
mit Aminen der Formel (IV)

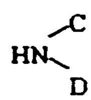 (IV)

in welcher
C und D die oben angegebene Bedeutung haben,
gegebenenfalls in Gegenwart eines Verdünnungsmittels und gegebenenfalls in Gegenwart eines wasserentziehenden Mittels umsetzt.

Schließlich wurde gefunden, daß die neuen 4-Alkoxy- bzw. 4-(substituierten)Amino-3-arylpyrrolinone der allgemeinen Formel (I) herbizide Eigenschaften besitzen.

Überraschenderweise zeigen die erfindungsgemäßen 4-Alkoxy- bzw. 4-(substituierten)Amino-3-arylpyrrolinone der allgemeinen Formel (I) eine hervorragende herbizide Wirksamkeit und gleichzeitig eine ausgezeichnete Verträglichkeit gegenüber wichtigen Kulturpflanzen.

Besonders bevorzugt ist die Gruppe von Verbindungen der Formel (I), bei welchen

B für Hydroxy, Methoxy, Ethoxy, n- oder iso-Propoxy, n-, iso-, sec.- oder tert.-Butoxy, Halogen-$C_1$-$C_4$-alkoxy mit Fluor und/oder Chloratomen oder Cycloalkoxy mit 3 bis 6 Ringatomen steht, wobei der Cycloalkyl-Rest unsubstituiert oder einfach bis dreifach, gleich oder verschieden durch Fluor, Chlor, Methyl und Ethyl substituiert ist,

X für Wasserstoff, Fluor, Chlor, Methyl, Ethyl, n- oder iso-Propyl, Methoxy, Ethoxy, n- oder i-Propoxy, Halogenmethyl mit 1, 2 oder 3 Fluor- und/oder Chloratomen, Halogenethyl mit 1 bis 5, insbesondere 1 bis 3, Fluor- und/oder Chloratomen, jeweils unsubstituiertes oder einfach bis fünffach, insbesondere einfach bis dreifach, gleich oder verschieden substituiertes Phenoxy oder Phenylthio, wobei als Phenylsubstituenten ausgewählt sind: Fluor, Chlor, Brom, Methyl, Ethyl, n- oder iso-Propyl, Methoxy, Ethoxy, n- oder iso-Propoxy und Trifluormethyl,

Y für Wasserstoff, Fluor, Chlor, Methyl, Ethyl, Methoxy, Ethoxy oder Trifluormethyl steht,

Z für Fluor oder Chlor steht,

n für 0, 1, 2 oder 3 steht und

A für Methyl, Ethyl, jeweils geradkettiges oder verzweigtes Propyl, Butyl, Pentyl, Hexyl, Heptyl, Octyl, Nonyl oder Decyl, Halogen-$C_1$-$C_6$-alkyl, $C_3$-$C_6$-Alkenyl, $C_3$-$C_6$-Alkinyl, $C_1$-$C_4$-Alkoxy-$C_1$-$C_4$-alkyl, $C_1$-$C_4$-Alkylthio-$C_1$-$C_4$-alkyl steht oder für Cycloalkyl mit 3 bis 6 Ringatomen steht, das durch Sauerstoff, Stickstoff oder Schwefel unterbrochen sein kann, oder A für jeweils unsubstituiertes oder einfach bis fünffach, insbesondere einfach bis dreifach, gleich oder verschieden substituiertes Phenyl, Benzyl oder Phenethyl steht, wobei als Substituenten jeweils ausgewählt sind: Fluor, Chlor, Methyl, Ethyl, n-oder iso-Propyl, Methoxy, Ethoxy, Trifluormethyl, Trifluormethoxy oder jeweils unsubstituiertes oder einfach bis dreifach, gleich oder verschieden, durch Fluor, Chlor, Methyl, Methoxy, Trifluormethyl und Trifluormethoxy substituiertes Phenoxy oder Phenylthio,

mit der Maßgabe, daß wenigstens ein Rest X, Y oder Z nicht für Chlor oder Methyl steht, wenn B für Hydroxy und A eine andere Bedeutung als gegebenenfalls substituiertes Phenyl hat.

Besonders bevorzugt ist auch die Gruppe von Verbindungen der Formel (I), bei welchen

B für die Gruppe

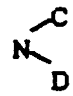

steht, wobei

C für Wasserstoff, Methyl, Ethyl, n- oder iso-Propyl steht und

D für Methyl, Ethyl, n- oder iso-Propyl, n-, iso-, sec.- oder tert.-Butyl, Allyl, Propargyl, Methoxymethyl, Methoxyethyl, Ethoxymethyl, Ethoxyethyl, Methylthiomethyl, Methylthioethyl, Ethylthiomethyl, Ethylthioethyl, Cyclopropyl, Cyclopentyl, Cyclohexyl, jeweils unsubstituiertes oder einfach bis fünffach, insbesondere einfach bis dreifach, gleich oder verschieden durch Fluor, Chlor, Methyl, Ethyl und Trifluormethyl substituiertes Phenyl, Benzyl oder Phenethyl, für Pyridyl oder Pyrimidyl steht oder

C und D zusammen mit dem Stickstoff, an den sie gebunden sind, einen 5- oder 6-gliedrigen Ring bilden, der ein oder zwei Doppelbindungen enthalten kann oder der ein weiteres Stickstoff-, Sauerstoff- oder Schwefelatom enthalten kann und der einfach bis dreifach, gleich oder verschieden durch Methyl, Ethyl, Methylcarbonyl und Ethylcarbonyl substituiert sein kann,

X für Wasserstoff, Fluor, Chlor, Methyl, Ethyl, n- oder iso-Propyl, Methoxy, Ethoxy, n- oder i-Propoxy, Halogenmethyl mit 1, 2 oder 3 Fluor- und/oder Chloratomen, Halogenethyl mit 1 bis 5, insbesondere 1 bis 3, Fluor- und/oder Chloratomen, jeweils unsubstituiertes oder einfach bis fünffach, insbesondere einfach bis dreifach, gleich oder verschieden substituiertes Phenoxy oder Phenylthio, wobei als Phenylsubstituenten ausgewählt sind: Fluor, Chlor, Brom, Methyl, Ethyl, n- oder iso-Propyl, Methoxy, Ethoxy, n- oder iso-Propoxy und Trifluormethyl,

Y für Wasserstoff, Fluor, Chlor, Methyl, Ethyl, Methoxy, Ethoxy oder Trifluormethyl steht,

Z für Fluor oder Chlor steht,

n für 0, 1, 2 oder 3 steht, und

A für Methyl, Ethyl, jeweils geradkettiges oder verzweigtes Propyl, Butyl, Pentyl, Hexyl, Heptyl, Octyl, Nonyl oder Decyl, Halogen-$C_1$-$C_6$-alkyl, $C_3$-$C_6$-Alkenyl, $C_3$-$C_6$-Alkinyl, $C_1$-$C_4$-Alkoxy-$C_1$-$C_4$-alkyl, $C_1$-$C_4$-Alkylthio-$C_1$-$C_4$-alkyl steht oder für Cycloalkyl mit 3 bis 6 Ringatomen steht, das durch Sauerstoff, Stickstoff oder Schwefel unterbrochen sein kann, oder A für jeweils unsubstituiertes oder einfach bis fünffach, insbesondere einfach bis dreifach, gleich oder verschieden substituiertes Phenyl, Benzyl oder Phenethyl steht, wobei als Substituenten jeweils ausgewählt sind: Fluor, Chlor, Methyl, Ethyl, n-oder iso-Propyl, Methoxy, Ethoxy, Trifluormethyl, Trifluormethoxy oder jeweils unsubstituiertes oder einfach bis dreifach, gleich oder verschieden, durch Fluor, Chlor, Methyl, Methoxy, Trifluormethyl und Trifluormethoxy substituiertes Phenoxy oder Phenylthio.

Ganz besonders bevorzugt ist die Gruppe von Verbindungen, in denen

B für Hydroxy, Methoxy, Ethoxy, n- oder iso-Propoxy steht,

X für Fluor, Chlor oder Trifluormethyl in meta-Position des Phenylringes oder für jeweils unsubstituiertes oder einfach bis dreifach, gleich oder verschieden substituiertes Phenyl oder Phenoxy steht, wobei als Substituenten ausgewählt sind: Fluor, Chlor, Methyl und Trifluormethyl,

Y für Wasserstoff, Fluor oder Chlor steht,

n für 0 steht und

A für Methyl, Ethyl, n-Propyl, iso-Propyl, n-, iso-, sec.- oder tert.-Butyl, jeweils geradkettiges oder verzweigtes Pentyl, Hexyl, Heptyl oder Octyl, Halogen-$C_1$-$C_3$-alkyl, Allyl, Propargyl, Methoxymethyl, Methoxyethyl, Ethoxymethyl, Ethoxyethyl, Cyclopropyl, Cyclopentyl, Cyclohexyl, jeweils unsubstituiertes oder einfach bis dreifach, gleich oder verschieden durch Fluor, Chlor, Trifluormethyl, Phenoxy und 4-Trifluormethyl-phenoxy substituiertes Phenyl oder Benzyl steht.

Ganz besonders bevorzugt ist auch die Gruppe von Verbindungen der Formel (I), in welcher

B für die Gruppe

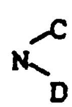

steht, wobei

C für Wasserstoff steht und

D für Methyl, Ethyl, n- oder iso-Propyl, n-, iso-, sec.- oder tert.-Butyl, unsubstituiertes oder einfach bis dreifach, gleich oder verschieden durch Fluor, Chlor und Trifluormethyl substituiertes Phenyl steht oder

C und D zusammen mit dem Stickstoff, an den sie gebunden sind, für einen gegebenenfalls einfach bis dreifach, gleich oder verschieden durch Methyl, Ethyl, Methylcarbonyl und Ethylcarbonyl substituierten Heterocyclus der Formel stehen:

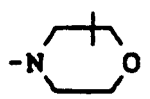 , 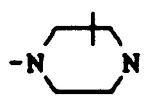 , 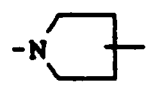 , 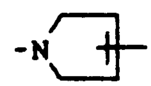

X für Fluor, Chlor oder Trifluormethyl in meta-Position des Phenylringes oder für jeweils unsubstituiertes oder einfach bis dreifach, gleich oder verschieden substituiertes Phenyl oder Phenoxy steht, wobei als Substituenten ausgewählt sind: Fluor, Chlor, Methyl und Trifluormethyl,

Y für Wasserstoff, Fluor oder Chlor steht,

n für 0 steht und

A für Methyl, Ethyl, n-Propyl, iso-Propyl, n-, iso-, sec.- oder tert.-Butyl, jeweils geradkettiges oder verzweigtes Pentyl, Hexyl, Heptyl oder Octyl, Halogen-$C_1$-$C_3$-alkyl, Allyl, Propargyl, Methoxymethyl, Methoxyethyl, Ethoxymethyl, Ethoxyethyl, Cyclopropyl, Cyclopentyl, Cyclohexyl, jeweils unsubstituiertes oder einfach bis dreifach, gleich oder verschieden durch Fluor, Chlor, Trifluormethyl, Phenoxy und 4-Trifluormethyl-phenoxy substituiertes Phenyl oder Benzyl steht.

In allen oben aufgeführten Definitionsbereichen ist besonders die Bedeutung von X als Trifluormethyl in der meta-Position des Phenylringes hervorzuheben.

Verwendet man gemäß dem allgemeinen Herstellungsverfahren (a) N-(3-Trifluormethylphenylacetyl-N-methylaminoessigsäureethylester, so kann der Verlauf des erfindungsgemäßen Verfahrens durch folgendes Reaktionsschema wiedergegeben werden:

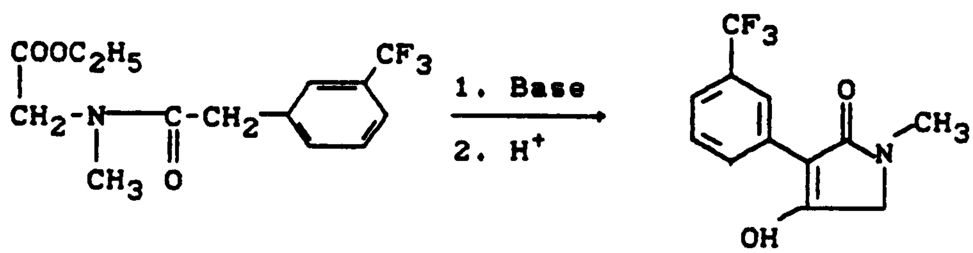


Verwendet man gemäß allgemeinem Herstellungsverfahren b) 3-(3-Trifluormethylphenyl)-1-tert.-butyl-pyrrolidin-2,4-dion und p-Toluolsulfonsäuremethylester als Ausgangsstoffe, so kann der Verlauf des erfindungsgemäßen Verfahrens durch folgendes Reaktionsschema wiedergegeben werden:

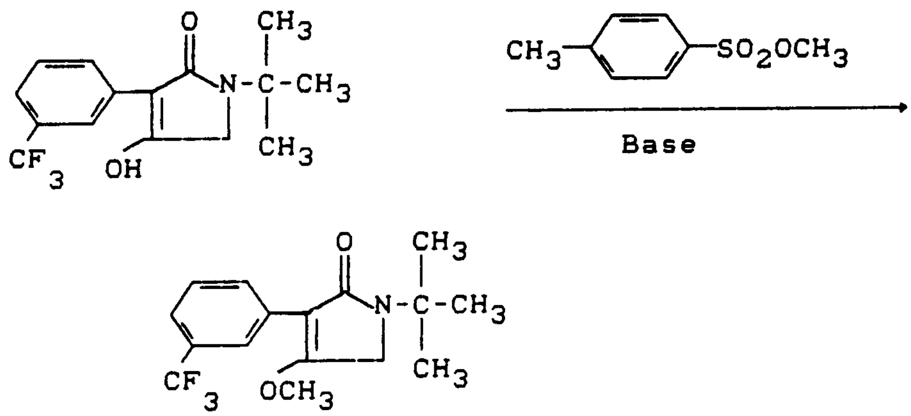


Verwendet man gemäß allgemeinem Herstellungsverfahren (c) 3-(3-Trifluormethylphenyl)-1-isopropyl-pyrrolidin-2,4-dion und Methylamin als Ausgangsstoffe, so kann der Verlauf des erfindungsgemäßen Verfahrens durch folgendes Reaktionsschema wiedergegeben werden:

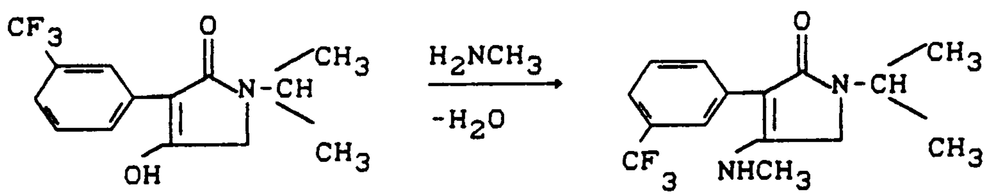


Die zur Durchführung des erfindungsgemäßen Verfahrens (a) als Ausgangsstoffe benötigten Verbindungen der Formel (II) sind teilweise bekannt oder lassen sich nach im Prinzip bekannten Methoden in einfacher Weise herstellen.

So erhält man beispielsweise N-Acylaminosäureester der Formel (II), wenn man Acylaminosäureester der Formel (V)

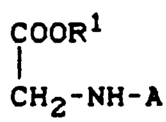

(V)

in welcher
- $R^1$ für Wasserstoff oder Alkyl, insbesondere Methyl oder Ethyl, steht und
- A die oben angegebene Bedeutung hat,

mit Phenylessigsäurehalogeniden der Formel (VI)

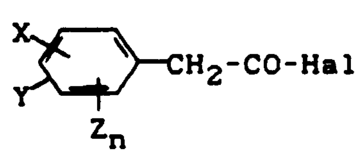

(VI)

in welcher
- Hal für Halogen, insbesondere Fluor, Chlor oder Brom, steht und
- X, Y, Z und n die oben angegebene Bedeutung haben,

in üblicher Weise acyliert (vgl. Chem. Rev. 52 (1953) 237-416 und Organikum, 9. Auflage 446 (1970) VEB Deutscher Verlag der Wissenschaften)
oder wenn man N-Acylaminosäuren der Formel (II), in welcher $R^1$ für Wasserstoff steht, in üblicher Weise

verestert (vgl. Chem. Ind. (London) 1568 (1968)).

Verbindungen der Formel (II), in denen $R^1$ für Wasserstoff steht, lassen sich beispielsweise auch aus Phenylessigsäurehalogeniden der Formel (VI) und Aminosäuren herstellen.

Verbindungen der Formel (V) sind nach literaturbekannten Verfahren aus $\alpha$-Halogencarbonsäuren bzw. -estern und Aminen erhältlich (Advanced Organic Chemistry, J. March S. 377, Mc Graw-Hill Inc (1977)).

Die Phenylessigsäurehalogenide der Formel (VI) sind bekannte Verbindungen der organischen Chemie.

Beispielhaft sind folgende Verbindungen der Formel (II) genannt:

1. N-(3-Trifluormethylphenylacetyl)-N-isopropylaminoessigsäureethylester
2. N-(3-Trifluormethylphenylacetyl)-N-(1,1,3,3-tetramethyl)butyl-aminoessigsäureethylester
3. N-(3-Trifluormethylphenylacetyl)-N-2,4,6-trimethylphenyl-aminoessigäsureethylester
4. N-(3-Trifluormethylphenylacetyl)-N-cyclobutyl-aminoessigsäureethylester
5. N-(3-Trifluormethylphenylacetyl)-N-cyclopentylaminoessigsäureethylester
6. N-(3-Trifluormethylphenylacetyl)-N-cyclohexyl-aminoessigsäureethylester
7. N-(3-Trifluormethylphenylacetyl)-N-(2-methoxyethylaminoesigsäureethylester
8. N-(3-Trifluormethylphenylacetyl)-N-(1-trifluormethyl-ethyl)aminoessigsäureethylester
9. N-(3-Trifluormethylphenylacetyl)-N-phenyl-aminoessigsäureethylester
10. N-(3-Trifluormethylphenylacetyl)-N-2-chlorphenylaminoessigsäureethylester
11. N-(3-Trifluormethylphenylacetyl)-N-4-chlorphenylaminoessigsäureethylester
12. N-(3-Trifluormethylphenylacetyl)-N-2,4-dichlorphenyl-aminoessigsäureethylester
13. N-(3-Trifluormethylphenylacetyl)-N-2,6-dichlorphenyl-aminoessigsäureethylester
14. N-(3-Trifluormethylphenylacetyl)-N-4-trifluormethylphenyl-aminoessigsäureethylester
15. N-(3-Trifluormethylphenylacetyl)-N-2-chlor-5-trifluormethylphenyl-aminoessigsäureethylester
16. N-(3-Trifluormethylphenylacetyl)-N-5-trifluormethylphenyl-aminoessigsäureethylester
17. N-(3-Trifluormethylphenylacetyl)-N-2-fluorphenylaminoessigsäureethylester
18. N-(3-Trifluormethylphenylacetyl)-N-4-fluorphenylaminoessigsäureethylester
19. N-(3-Trifluormethylphenylacetyl)-N-2,6-difluorphenyl-aminoessigsäureethylester
20. N-(3-Trifluormethylphenylacetyl)-N-2,4-difluorphenyl-aminoessigsäureethylester
21. N-(3-Trifluormethylphenylacetyl)-N-2,2-dimethylpropyl-aminoessigsäureethylester
22. N-(3-Trifluormethylphenylacetyl)-N-1,2,2-trimethylpropyl-aminoessigsäureethylester
23. N-(3-Trifluormethylphenylacetyl)-N-benzyl-aminoessigsäureethylester
24. N-Phenylacetyl-N-(3-trifluormethylphenyl-aminoessigsäureethylester
25. N-Phenylacetyl-N-cyclopropyl-aminoessigsäureethylester
26. N-(3-Trifluormethylphenylacetyl)-N-1,2-dimethylpropyl-aminoessigsäureethylester
27. N-(3-Trifluormethylphenylacetyl)-N-3,4-dichlorphenyl-aminoessigsäureethylester
28. N-(2,4,6-Trichlor-3-fluor-5-phenoxyphenylacetyl)-N-phenyl-aminoessigsäureethylester
29. N-(4-Trifluormethyl-2-chlor-6-fluorphenylacetyl)-N-phenyl-aminoessigsäureethylester
30. N-(3-Trifluormethylphenylacetyl)-N-3-chlorphenylaminoessigsäureethylester
31. N-Phenylacetyl-N-phenyl-aminoessigsäureethylester
32. N-Phenylacetyl-N-2,4-difluorphenyl-aminoessigsäureethylester
33. N-(2-(2,4-Dimethylphenoxy)phenylacetyl)-N-phenyl-aminoessigsäureethylester
34. N-(2-Phenoxy-phenylacetyl)-N-phenyl-aminoessigsäureethylester
35. N-(3-Trifluormethylphenylacetyl)-N-4-phenoxyphenyl)-aminoessigsäureethylester
36. N-(3-Trifluormethylphenylacetyl)-N-4-(4-trifluormethylphenoxy)phenyl-aminoessigsäureethylester
37. N-(4-Trifluormethyl-2-chlor-6-fluorphenylacetyl)-N-phenyl-aminoessigsäureethylether
38. N-Phenyl-N-2-chlorphenyl-aminoessigsäurephenylester
39. N-(2,4-Difluorphenylacetyl)-N-3-trifluormethylphenyl-aminoessigsäureethylester
40. N-(3-Trifluormethylphenyl)-N-(3-trifluormethylphenyl-4-phenoxyphenyl)-aminoessigsäureethylester
41. Ethyl-N-phenylacetyl-N-3-chlorophenylaminoacetate
42. Ethyl-N-phenylacetyl-N-4-chlorophenylaminoacetate
43. Ethyl-N-phenylacetyl-N-3,4-dichlorophenylaminoacetate
44. Ethyl-N-(3-trifluoromethylphenylacetyl)-N-methylaminoacetate
45. Ethyl-N-(3-trifluoromethylphenylacetyl)-N-tert.-butylaminoacetate
46. Ethyl-N-(3-phenoxy-phenylacetyl)-N-phenylaminoacetate
47. Ethyl-N-(3-phenoxy-phenylacetyl)-N-(3-trifluoromethylphenyl)-aminoacetate
48. Ethyl-N-(4-phenoxy-phenylacetyl)-N-phenylaminoacetate
49. Ethyl-N-(4-phenoxy-phenylacetyl)-N-(trifluoromethylphenyl)-aminoacetate
50. Ethyl-N-(3-trifluromethylphenylacetyl)-N-(4-cyclohexylphenyl)-aminoacetate
51. Ethyl-N-(2-phenoxy-phenylacetyl)-N-(3-trifluoromethylphenyl)-aminoacetate

52. Ethyl-N-phenylacetyl-N-(4-flurophenyl)-aminoacetate
53. Ethyl-N-(3-trifluoromethylphenylacetyl)-N-(2-methylphenyl)-aminoacetate
54. Ethyl-N-(3-trifluoromethylphenylacetyl)-N-(3-methylphenyl)-aminoacetate
55. Ethyl-N-(3-trifluoromethylphenylacetyl)-N-(4-methylphenyl)-aminoacetate
56. Ethyl-N-(4-methylphenylacetyl)-N-(4-fluorophenyl)-aminoacetate
57. Ethyl-N-(2-methylphenylacetyl)-N-(4-fluorophenyl)-aminoacetate
58. Ethyl-N-(2-methylphenylacetyl)-N-(3-trifluoromethylphenyl)-aminoacetate
59. Ethyl-N-(2-fluorophenylacetyl)-N-(2-methylphenyl)-aminoacetate
60. Ethyl-N-(4-fluorophenylacetyl)-N-(2-methylphenyl)-aminoacetate
61. Ethyl-N-(2-fluorophenylacetyl)-N-(4-methylphenyl)-aminoacetate
62. Ethyl-N-(2-fluorophenylacetyl)-N-(3-trifluoromethylphenyl)-aminoacetate
63. Ethyl-N-(4-fluorophenylacetyl)-N-phenylaminoacetate
64. Ethyl-N-(4-fluorophenylacetyl)-N-(3-chlorophenyl)-aminoacetate
65. Ethyl-N-(2,4-difluorophenylacetyl)-N-(4-fluorophenyl)-aminoacetate
66. Ethyl-N-(2,4-difluorophenylacetyl)-N-(2-methylphenyl)-aminoacetate
67. Ethyl-N-(2,4-difluorophenylacetyl)-N-(3-methylphenyl)-aminoacetate
68. Ethyl-N-(2,4-difluorophenylacetyl)-N-(4-methylphenyl)-aminoacetate
69. Ethyl-N-(2,4-difluorophenylacetyl)-N-(2,4-difluorophenyl)-aminoacetate
70. Ethyl-N-(3-fluorophenylacetyl)-N-(3-methylphenyl)-aminoacetate
71. Ethyl-N-(3-fluorophenylacetyl)-N-(4-methylphenyl)-aminoacetate
72. Ethyl-N-(3-fluorophenylacetyl)-N-(3-fluorophenyl)-aminoacetate
73. Ethyl-N-(2-methylphenylacetyl)-N-phenylaminoacetate

Die zur Durchführung des erfindungsgemäßen Verfahrens (b) und (c) als Ausgangsstoffe benötigten Verbindungen der Formel (I) mit B = Hydroxyl sind erfindungsgemäße Verbindungen und erhältlich nach Verfahren (a).

Die weiterhin zur Durchführung des erfindungsgemäßen Verfahrens (b) als Ausgangsstoffe benötigten Alkylierungsreagenzien der Formel (III) sind bekannte Verbindungen der organischen Chemie.

In der Formel (III) steht $R^2$ vorzugsweise für $C_1$-$C_{10}$-Alkyl, Halogen-$C_1$-$C_{10}$-alkyl oder Cycloalkyl mit 3 bis 7 Ringatomen, wobei der Cycloalkyl-Rest unsubstituiert oder einfach bis fünffach, gleich oder verschieden durch Fluor, Chlor, Brom und $C_1$-$C_4$-Alkyl substituiert ist. Besonders bevorzugt steht $R^2$ für Methyl, Ethyl, Propyl oder iso-Propyl.

In der Formel (III) steht $X^1$ bevorzugt für Chlor, Brom oder Iod, $C_1$-$C_4$-Alkylsulfonyloxy, $C_1$-$C_4$-Alkoxysulfonyloxy oder p-Toluolsulfonyloxy, wie beispielsweise Methansulfonyloxy, Methoxysulfonyloxy oder Ethoxysulfonyloxy.

Die Alkylierungsreagenzien der Formel (III) sind bekannte Verbindungen der organischen Chemie.

Die zur Durchführung des erfindungsgemäßen Verfahrens (c) als Ausgangsstoffe benötigten Amine der Formel (IV) sind allgemein bekannte Verbindungen oder lassen sich nach bekannten Methoden herstellen (vgl. Houben-Weyl, Methoden der Organischen Chemie, Bd. XI/1, Georg-Thieme-Verlag, Stuttgart 1957).

Das Verfahren (a) ist dadurch gekennzeichnet, daß man Verbindungen der Formel (II) in Gegenwart von Basen einer intramolekularen Kondensation unterwirft.

Als Verdünnungsmittel können bei dem erfindungsgemäßen Verfahren (a) alle üblichen inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glylkoldimethylether und Diglykoldimethylether, außerdem polare Lösungsmittel, wie Dimethylsulfoxid, Sulfolan, Dimethylformamid und N-Methylpyrrolidon.

Als Deprotonierungsmittel können bei der Durchführung des erfindungsgemäßen Verfahrens (a) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat und Kalium-tert.-butylat einsetzbar.

Die Reaktionstemperaturen können bei der Durchführung des erfindungsgemäßen Verfahrens (a) innerhalb eines größeren Bereiches variiert werden. Im allgemeinen arbeitet man bei Temperaturen

zwischen 0 °C und 250 °C, vorzugsweise zwischen 50 °C und 150 °C. Das erfindungsgemäße Verfahren (a) wird im allgemeinen unter Normaldruck durchgeführt.

Bei der Durchführung des erfindungsgemäßen Verfahrens (a) setzt man die Reaktionskomponenten der Formel (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Zur Durchführung des erfindungsgemäßen Verfahrens (b) kommen als Verdünnungsmittel inerte organische Lösungsmittel infrage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Säuren, wie Essigsäure, Nitrile, wie Acetonitril oder Propionitril, Amide, wie Dimethylformamid, Dimethylacetamid, N-Methyl-pyrrolidon oder Hexamethylphosphorsäuretriamid. Verwendet man als Reaktionspartner Verbindungen der Formel (III) in flüssiger Form, so ist es auch möglich, diese in entsprechendem Überschuß gleichzeitig als Verdünnungsmittel einzusetzen.

Als Säurebindemittel zur Durchführung des erfindungsgemäßen Verfahrens (b) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (b) setzt man pro Mol an Verbindung der Formel (I), in der B für Hydroxy steht, im allgemeinen jeweils 1 bis 20 Mol, vorzugsweise jeweils 1 bis 15 Mol, an Alkylierungsmittel der Formel (III) und gegebenenfalls 1 bis 3 Mol, vorzugsweise 1 bis 2 Mol an Säurebindemittel ein. Die Reaktionsdurchführung, Aufarbeitung und Isolierung der Reaktionsprodukte der Formel (I) erfolgt nach üblichen Methoden (vgl. auch die Herstellungsbeisiele).

Als Verdünnungsmittel zur Durchführung des erfindungsgemäßen Verfahrens (c) kommen vorzugsweise inerte organische Lösungsmittel in Frage. Hierzu gehören insbesondere aliphatische, alicyclische oder aromatische, gegebenenfalls halogenierte Kohlenwasserstoffe, wie beispielsweise Benzin, Ligroin, Benzol, Toluol, Xylol, Chlorbenzol, Petrolether, Pentan, Hexan, Heptan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester. Die Amine der Formel (IV) in flüssiger Form können in entsprechendem Überschuß ebenfalls als Lösungsmittel eingesetzt werden.

Das erfindungsgemäße Verfahren (c) wird gegebenenfalls in Gegenwart eines wasserentziehenden Mittels durchgeführt. Vorzugsweise verwendet man als wasserentziehendes Mittel Alkalimetallcarbonate wie z.B. Kaliumcarbonat; Molekularsiebe oder - in katalytischen Mengen - z.B. p-Toluolsulfonsäure. Als günstige Verfahrensweise stellt sich das azeotrope Abdestillieren des Wassers während der Reaktion dar.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 250 °C, vorzugsweise bei Temperaturen zwischen 20 °C und 200 °C.

Das erfindungsgemäße Verfahren (c) wird üblicherweise unter Normaldruck durchgeführt. Es ist jedoch auch möglich unter erhöhtem Druck zu arbeiten.

Zur Durchführung des erfindungsgemäßen Verfahrens c) setzt man pro Mol an Verbindung der Formel (I) in der B für Hydroxy steht, im allgemeinen 1 bis 10 Mol, vorzugsweise 1 bis 2,5 Mol an Amin der Formel (IV) und gegebenenfalls 1 bis 5 Mol an wasserentziehendem Mittel ein. Die Reaktionsdurchführung, Aufarbeitung und Isolierung der Reaktionsprodukte erfolgt in Analogie zu allgemein bekannten Verfahren.

Adogen 464 = Methyltrialkyl($C_8$-$C_{10}$)ammoniumchlorid

TDA 1 = Tris-(methoxyethoxylethyl)-amin

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich sehr gut zur selektiven Bekämpfung von mono- und dikotylen Unkräutern in mono- und dikotylen Kulturen im Vor- und Nachauflaufverfahren.

Darüber hinaus zeigen die erfindungsgemäßen Verbindungen auch eine blattinsektizide Wirkung.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von

Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. Aryloxyalkansäuren, wie 2,4 D, 2,4 DP, 2,4-DB, MCPA, MCPP, Fluoroxypyr, Aryloxy-phenyl-alkansäureester, wie Diclofop-methyl, Fenoxaprop, Fluazifop-butyl, Quizalofop, Haloxyfop, Arylcarbonsäuren, wie Clopyralid, Azinone, wie Chloridazon, Norflurazon, Carbamate, wie Phenmedipham, Propham, Chlorpropham, Asulam, Chloracetanilide, wie Alachlor, Butachlor, Metazachlor, Metolachlor, Pretilachlor, Propachlor, Dinitroaniline, wie Oryzalin, Pendimethalin, Trifluralin, Diphenylether wie Acifluorfen, Bifenox, Fomesafen, Lactofen, Oxyfluorfen, Harnstoffe, wie Chlortoluron, Fluormeturon, Isoproturon, Methabenzthiazuron, Hydroxylamine, wie Alloxydim, Cycloxydim, Sethoxydim, Imidazolinone, wie Imazethapyr, Imazamethabenz, Imazaquin, Nitrile, wie Bromoxynil, Ioxynil, Oxyacetamide, wie Mefenacet, Sulfonylharnstoffe, wie Bensulfuron, Chlorimuron, Chlorsulfuron, Metsulfuron, Thiameturon, Thiolcarbamate, wie Cycloate, EPTC, Molinate, Thiobencarb, Triallate, Triazindione, wie Amethydione, Triazine, wie Atrazin, Cyanazin, Simazin, Simetryne, Terbutryne oder Triazine, wie Ethiozin, Hexazinon, Metamitron, Metribuzin, oder Sonstige Bentazone, Cinmethylin, Fluridone, Glyphosate, Pyridate, Dimethazone in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,01 und 5 kg pro ha.

Die Herstellung und die Verwendung der erfindungsgemäßen Wirkstoffe geht aus den nachfolgenden Beispielen hervor.

Herstellungsbeispiele

Beispiel 1

Verfahren (a)

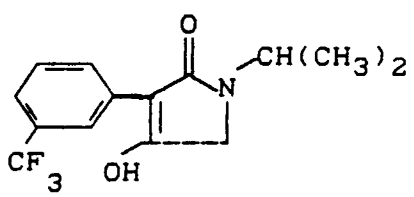

3-(3-Trifluormethylphenyl)-1-isopropyl-pyrrolidin-2,4-dion

Zu 3,6 g (0,12 Mol) Natriumhydrid, 80 %ig, in 55 ml absolutem Toluol werden bei 95 bis 100 °C 33,1 g (0,1 Mol) N-(3-Trifluormethylphenylacetyl)-N-isopropyl-aminoessigsäureethylester in 100 ml absolutem Toluol in ca. 60 Minuten zugetropft. Anschließend wird eine weitere Stunde bei 100 °C nachgerührt, dann unter Eisbadkühlung 30 ml Ethanol, anschließend 80 ml Wasser, gefolgt von 10 ml konzentrierter Salzsäure, zugetropft. Das ausgefallene Produkt wird abgesaugt, zuerst mit Wasser, dann mit Petrolether gut nachgewaschen und getrocknet. Man erhält an farblosen Kristallen des Produktes:

51,1 g (78,2 % der Theorie), Fp. 207 bis 208 °C.

In analoger Weise zu Beispiel 1 und unter Berücksichtigung der Angaben in der Beschreibung zum erfindungsgemäßen Verfahren (a) erhält man die nachfolgend in Tabelle 1 aufgeführten Endprodukte der Formel (I), in denen B für Hydroxy steht:

**Tabelle 1**

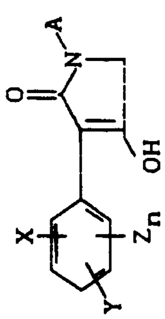

(Ia)

| Bsp.Nr. | X | Y | $Z_n$ | A | Schmelzpunkt/°C |
|---|---|---|---|---|---|
| 2 | $3-CF_3$ | H | - | $-C(CH_3)_2-CH_2-C(CH_3)_3$ | 223 |
| 3 | $3-CF_3$ | H | - | 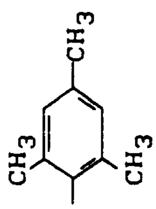 | 248 |
| 4 | $3-CF_3$ | H | - | 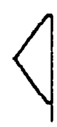 | 240 (Zers.) |
| 5 | $3-CF_3$ | H | - | 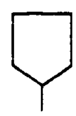 | 234 |
| 6 | $3-CF_3$ | H | - | 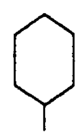 | 245 |
| 7 | $3-CF_3$ | H | - | $-CH_2CH_2-O-CH_3$ | 181 |

Tabelle 1 (Fortsetzung)

| Bsp.Nr. | X | Y | $Z_n$ | A | Schmelzpunkt/°C |
|---|---|---|---|---|---|
| 8 | $3-CF_3$ | H | - | 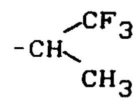 | 184 |
| 9 | $3-CF_3$ | H | - | 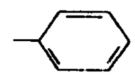 | 244 (Zers.) |
| 10 | $3-CF_3$ | H | - | 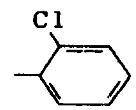 | 199 |
| 11 | $3-CF_3$ | H | - | 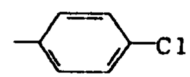 | 216 |
| 12 | $3-CF_3$ | H | - | 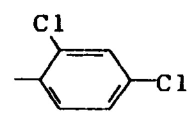 | 108 |

Tabelle 1 (Fortsetzung)

| Bsp.Nr. | X | Y | $Z_n$ | A | Schmelzpunkt/°C |
|---|---|---|---|---|---|
| 13 | 3-$CF_3$ | H | - | 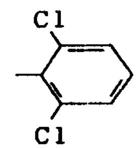 | 182 |
| 14 | 3-$CF_3$ | H | - | 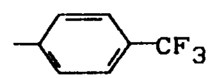 | 224 |
| 15 | 3-$CF_3$ | H | - | 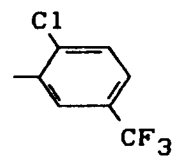 | 189 |
| 16 | 3-$CF_3$ | H | - | 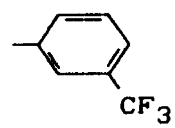 | 208 |
| 17 | 3-$CF_3$ | H | - | 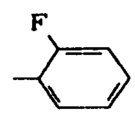 | 235 |

Tabelle 1 (Fortsetzung)

| Bsp.Nr. | X | Y | $Z_n$ | A | Schmelzpunkt/°C |
|---|---|---|---|---|---|
| 18 | 3-$CF_3$ | H | - | 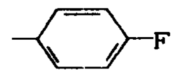 | 230 |
| 19 | 3-$CF_3$ | H | - | 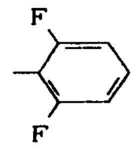 | Öl |
| 20 | 3-$CF_3$ | H | - | 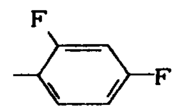 | 226 |
| 21 | 3-$CF_3$ | H | - | $-CH_2-C(CH_3)_3$ | 242 |
| 22 | 3-$CF_3$ | H | - | $-CH(CH_3)-C(CH_3)_3$ | 125 |
| 23 | 3-$CF_3$ | H | - | $-CH_2$ 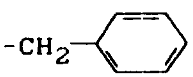 | 201 |
| 24 | H | H | - | 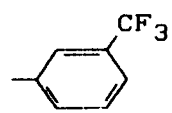 | ›250 |

**Tabelle 1** (Fortsetzung)

| Bsp.Nr. | X | Y | $Z_n$ | A | Schmelzpunkt/°C |
|---|---|---|---|---|---|
| 25 | H | H | - | 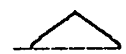 | 238 (Zers.) |
| 26 | $3-CF_3$ | H | - | $-CH(CH_3)-CH(CH_3)_2$ | 193 |
| 27 | $3-CF_3$ | H | - | 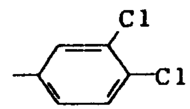 | 239 |
| 28 | 3-O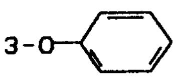 | 5-F | $2,4,6-Cl_3$ | 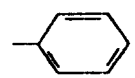 | 204 |
| 29 | 4-O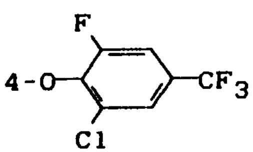 | H | - | 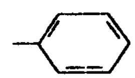 | >250 |

Tabelle 1 (Fortsetzung)

| Bsp.Nr. | X | Y | $Z_n$ | A | Schmelzpunkt/°C |
|---|---|---|---|---|---|
| 30 | 3-$CF_3$ | H | - | 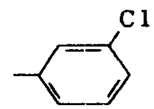 | 227 |
| 31 | H | H | - | 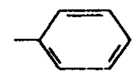 | 〉250 |
| 32 | H | H | - | 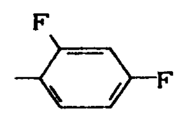 | 226 |
| 33 | 2-O 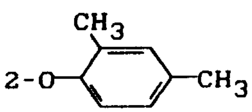 | H | - | 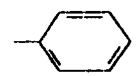 | 170 |
| 34 | 2-O 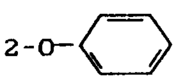 | H | - | 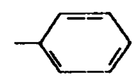 | 234 |
| 35 | 3-$CF_3$ | H | - | 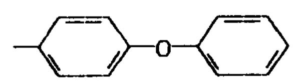 | 231 |

Tabelle 1 (Fortsetzung)

| Bsp.Nr. | X | Y | $Z_n$ | A | Schmelzpunkt/$^0$C |
|---|---|---|---|---|---|
| 36 | 3-$CF_3$ | H | - | 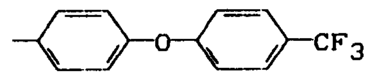 | 205 |
| 37 | 2-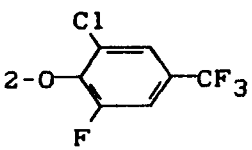 | H | - | 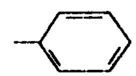 | 222 |
| 38 | H | H | - | 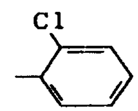 | 102 |
| 39 | 2-F | 4-F | - | 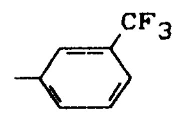 | 211 |
| 40 | 3-$CF_3$ | H | - | 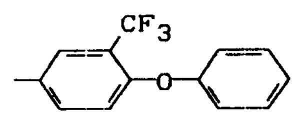 | Öl |

Tabelle 1 (Fortsetzung)

| Bsp.Nr. | X | Y | $Z_n$ | A | Schmelzpunkt/°C |
|---|---|---|---|---|---|
| 41 | H | H | - | 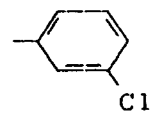 | 180 |
| 42 | H | H | - | 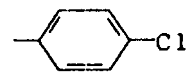 | 〉250 |
| 43 | H | H | - | 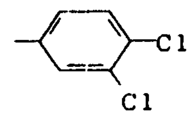 | 240 |
| 44 | 3-$CF_3$ | H | - | $CH_3$ | 260 |
| 45 | 3-$CF_3$ | H | - | $C(CH_3)_3$ | 〉230 |
| 46 | 3-O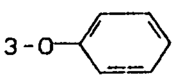 | H | - | 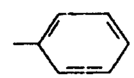 | 212-213 |
| 47 | 3-O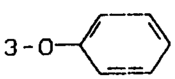 | H | - | 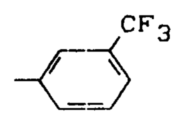 | 160 |

Tabelle 1 (Fortsetzung)

| Bsp.Nr. | X | Y | $Z_n$ | A | Schmelzpunkt/°C |
|---|---|---|---|---|---|
| 48 | 4-O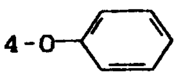 | H | - | 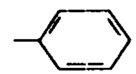 | 226-227 |
| 49 | 4-O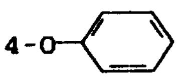 | H | - | 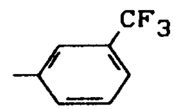 | 191 |
| 50 | 3-$CF_3$ | H | - | 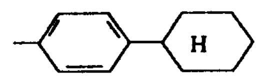 | 237-238 |
| 51 | 2-O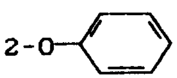 | H | - | 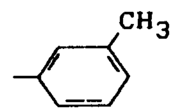 | 105 |
| 52 | H | H | - | 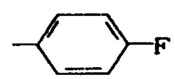 | > 250 |
| 53 | 3-$CF_3$ | H | - | 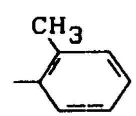 | 222-223 |

Tabelle 1 (Fortsetzung)

| Bsp.Nr. | X | Y | $Z_n$ | A | Schmelzpunkt/°C |
|---|---|---|---|---|---|
| 54 | 3-$CF_3$ | H | - | 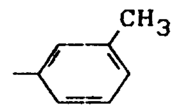 | 247-248 |
| 55 | 3-$CF_3$ | H | - | 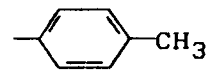 | ⟩ 250 |
| 56 | 4-$CH_3$ | H | - | 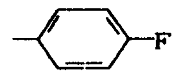 | ⟩ 250 |
| 57 | 2-$CH_3$ | H | - | 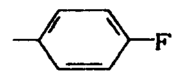 | 220-221 |
| 58 | 2-$CH_3$ | H | - | 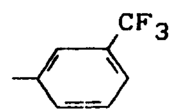 | Öl |
| 59 | 2-F | H | - | 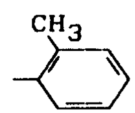 | 236-237 |

<u>Tabelle 1</u> (Fortsetzung)

| Bsp.Nr. | X | Y | $Z_n$ | A | Schmelzpunkt/°C |
|---|---|---|---|---|---|
| 60 | 4-F | H | - | 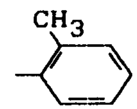 | › 250 |
| 61 | 2-F | H | - | 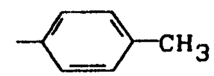 | › 250 |
| 62 | 2-F | H | - | 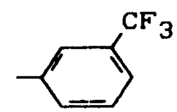 | 209 |
| 63 | 4-F | H | - | 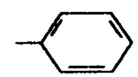 | › 250 |
| 64 | 4-F | H | - | 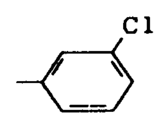 | 243-244 |
| 65 | $2,4-F_2$ | H | - | 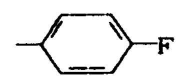 | 253-254 |

Tabelle 1 (Fortsetzung)

| Bsp.Nr. | X | Y | $Z_n$ | A | Schmelzpunkt/°C |
|---|---|---|---|---|---|
| 66 | 2,4-$F_2$ | H | - | 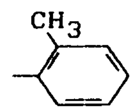 | 174 |
| 67 | 2,4-$F_2$ | H | - | 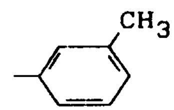 | 241 |
| 68 | 2,4-$F_2$ | H | - | 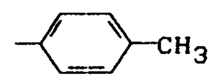 | > 250 |
| 69 | 2,4-$F_2$ | H | - | 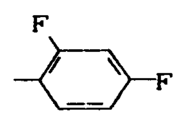 | 210-211 |
| 70 | 3-F | H | - | 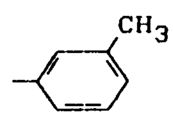 | 247-248 |
| 71 | 3-F | H | - | 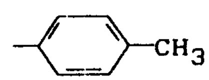 | 249-250 |

<u>Tabelle 1</u> (Fortsetzung)

| Bsp.Nr. | X | Y | $Z_n$ | A | Schmelzpunkt/°C |
|---|---|---|---|---|---|
| 72 | 3-F | H | - | 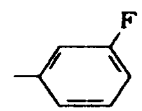 | 245-246 |
| 73 | 2-Me | H | - | 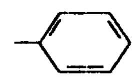 | 186 |

Beispiel 41a

Verfahren (c)

3-(3-Trifluormethylphenyl)-1-isopropyl-4-(N-methylamino)-3-pyrrolin-2-on

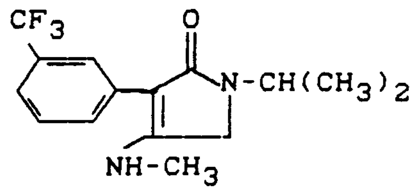

Zu einer Suspension von 28,5 g (0,1 Mol) 3-(3-Trifluormethylphenyl)-1-isopropyl-pyrrolidin-2,4-dion (hergestellt nach Beispiel 1) in 500 ml Xylol, der 50 ml Eisessig zugesetzt worden sind, werden 62,4 g einer 32 %igen wäßrigen Methylamin-Lösung gegeben. Das Gemisch wird 4 Stunden am Wasserabscheider erhitzt. Anschließend wird im Vakuum eingeengt, das zurückbleibende Rohprodukt in 300 ml Wasser aufgenommen, zweimal mit je 150 ml Dichlormethan extrahiert und die vereinigten Extrakte im Vakuum eingeengt. Das zurückbleibende Produkt mit Petrolether verrührt, abgesaugt und getrocknet. Man erhält 6,7 g (91 % der Theorie) des Produktes (farblose Kristalle) Fp.: 161-2 °C.

In analoger Weise zu Beispiel 41 und unter Berücksichtigung der Angaben in der Beschreibung zum erfindungsgemäßen Verfahren (c) erhält man die nachfolgend in Tabelle 2 aufgeführten Endprodukte der Formel (I), in denen

B für

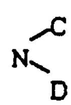

steht.

Tabelle 2

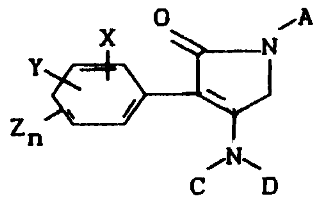

(Ib)

| Bsp.Nr. | X | Y | $Z_n$ | A | C | D | Schmelzpunkt/°C |
|---|---|---|---|---|---|---|---|
| 74 | $3-CF_3$ | H | - | $-C(CH_3)_2-CH_2-C(CH_3)_3$ | H | $CH_3$ | 132 |
| 75 | $3-CF_3$ | H | - | 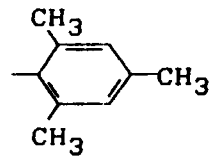 | H | $CH_3$ | 194 |
| 76 | $3-CF_3$ | H | - | 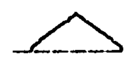 | H | $CH_3$ | 133 |
| 77 | $3-CF_3$ | H | - | 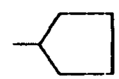 | H | $CH_3$ | 177 |
| 78 | $3-CF_3$ | H | - | 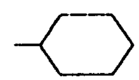 | H | $CH_3$ | 167 |
| 79 | $3-CF_3$ | H | - | $-CH_2CH_2-O-CH_3$ | H | $CH_3$ | Öl |

<u>Tabelle 2</u> (Fortsetzung)

| Bsp.Nr. | X | Y | $Z_n$ | A | C | D | Schmelzpunkt/°C |
|---|---|---|---|---|---|---|---|
| 80 | 3-$CF_3$ | H | - | 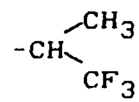 | H | $CH_3$ | 147 |
| 81 | 3-$CF_3$ | H | - | 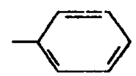 | H | $CH_3$ | 163 |
| 82 | 3-$CF_3$ | H | - | 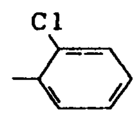 | H | $CH_3$ | 92 |
| 83 | 3-$CF_3$ | H | - | 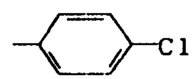 | H | $CH_3$ | 154 |
| 84 | 3-$CF_3$ | H | - | 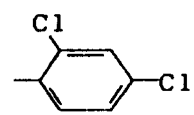 | H | $CH_3$ | 158 |

<u>Tabelle 2</u> (Fortsetzung)

| Bsp.Nr. | X | Y | $Z_n$ | A | C | D | Schmelzpunkt/°C |
|---|---|---|---|---|---|---|---|
| 85 | 3-$CF_3$ | H | - | 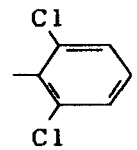 | H | $CH_3$ | 156 |
| 86 | 3-$CF_3$ | H | - | 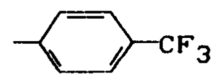 | H | $CH_3$ | 137 |
| 87 | 3-$CF_3$ | H | - | 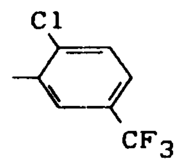 | H | $CH_3$ | 109 |
| 88 | 3-$CF_3$ | H | - | 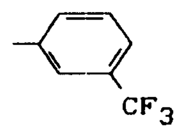 | H | $CH_3$ | 125 |
| 89 | 3-$CF_3$ | H | - | 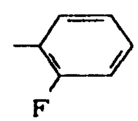 | H | $CH_3$ | 115 |

Tabelle 2 (Fortsetzung)

| Bsp.Nr. | X | Y | $Z_n$ | A | C | D | Schmelz-punkt/° C |
|---|---|---|---|---|---|---|---|
| 90 | $3-CF_3$ | H | - | 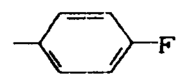 | H | $CH_3$ | 144 |
| 91 | $3-CF_3$ | H | - | 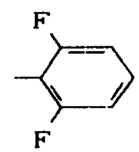 | H | $CH_3$ | 109 |
| 92 | $3-CF_3$ | H | - | 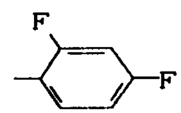 | H | $CH_3$ | 199 |
| 93 | $3-CF_3$ | H | - | $-CH(CH_3)_2$ | H | H | 166 |
| 94 | $3-CF_3$ | H | - | $-CH(CH_3)_2$ | H | $-CH(CH_3)_2$ | 162 |
| 95 | $3-CF_3$ | H | - | $-CH_3$ | H | $-CH(CH_3)_2$ | 158 |
| 96 | $3-CF_3$ | H | - | $-CH_2C(CH_3)_3$ | H | $CH_3$ | 168 |
| 97 | $3-CF_3$ | H | - | $-CH(CH_3)-C(CH_3)_3$ | H | $CH_3$ | 135 |

Tabelle 2 (Fortsetzung)

| Bsp.Nr. | X | Y | $Z_n$ | A | C | D | Schmelzpunkt/°C |
|---|---|---|---|---|---|---|---|
| 98 | $3-CF_3$ | H | - | $-CH_2$ 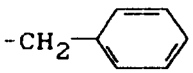 | H | $CH_3$ | 75 |
| 99 | H | H | - | 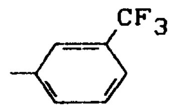 | H | $CH_3$ | 141 |
| 100 | $3-CF_3$ | H | - | $-CH(CH_3)_2$ | H | 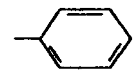 | 188 (Zers.) |
| 101 | $3-CF_3$ | H | - | 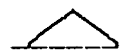 | H | 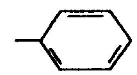 | 186 |
| 102 | $3-CF_3$ | H | - | 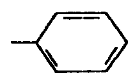 | H | 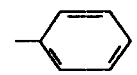 | 194 (Zers.) |
| 103 | H | H | - | 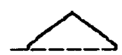 | H | 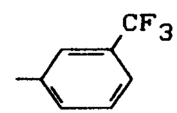 | 183 |
| 104 | $3-CF_3$ | H | - | $-CH(CH_3)-C(CH_3)_3$ | H | $CH_3$ | 118 |

Tabelle 2 (Fortsetzung)

| Bsp.Nr. | X | Y | $Z_n$ | A | C | D | Schmelzpunkt/°C |
|---|---|---|---|---|---|---|---|
| 105 | 3-$CF_3$ | H | - | 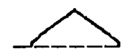 | H | H | 151 |
| 106 | 3-$CF_3$ | H | - | 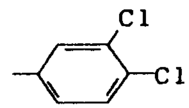 | H | $CH_3$ | 183 |
| 107 | 3-$CF_3$ | H | - | 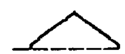 | H | 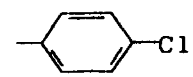 | 190 |
| 108 | 3-O 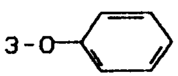 | 5-F | 2,4,6-$Cl_3$ | 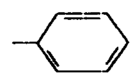 | H | $CH_3$ | 110 |
| 109 | 3-O 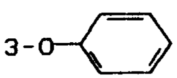 | 5-F | 2,4,6-$Cl_3$ | 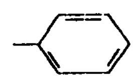 | H | 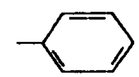 | 115 |
| 110 | 3-$CF_3$ | H | - | 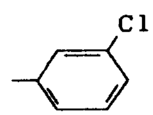 | H | $CH_3$ | 153 |

Tabelle 2 (Fortsetzung)

| Bsp.Nr. | X | Y | $Z_n$ | A | C | D | Schmelzpunkt/°C |
|---|---|---|---|---|---|---|---|
| 111 | 3-$CF_3$ | H | - | 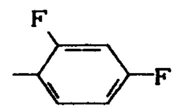 | H | $C_2H_5$ | 124 |
| 112 | H | H | - | 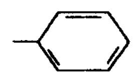 | H | 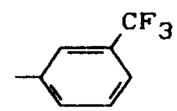 | 134 |
| 113 | H | H | - | 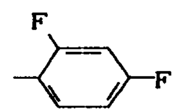 | H | $CH_3$ | 161 |
| 114 | H | H | - | 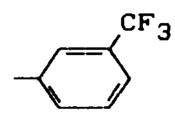 | H | 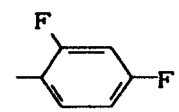 | >250 |
| 115 | 3-$CF_3$ | H | - | 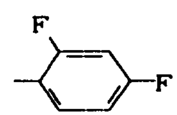 | | 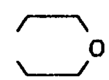 | 188 |

Tabelle 2 (Fortsetzung)

| Bsp.Nr. | X | Y | $Z_n$ | A | C | D | Schmelzpunkt/°C |
|---|---|---|---|---|---|---|---|
| 116 | 3-$CF_3$ | H | - | 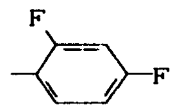 | | 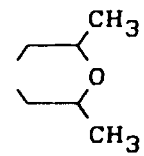 | 180 |
| 117 | 3-$CF_3$ | H | - | 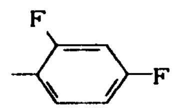 | | 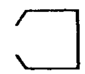 | 151 |
| 118 | 3-$CF_3$ | H | - | 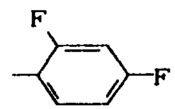 | | 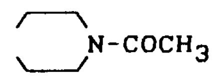 | >250 |
| 119 | 3-$CF_3$ | H | - | 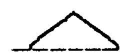 | | 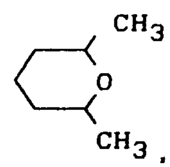, | 102 |
| 120 | 3-$CF_3$ | H | - | 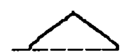 | | 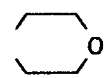 | 93 |

Tabelle 2 (Fortsetzung)

| Bsp.Nr. | X | Y | $Z_n$ | A | C | D | Schmelzpunkt/°C |
|---|---|---|---|---|---|---|---|
| 121 | 3-$CF_3$ | H | - | 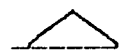 | | 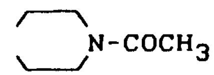 | 43 |
| 122 | 3-$CF_3$ | H | - | 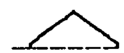 | | 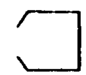 | 108 |
| 123 | 3-$CF_3$ | H | - | 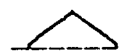 | | 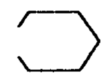 | 92 |
| 124 | 2-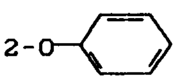 | H | - | 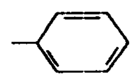 | H | $CH_3$ | 149 |
| 125 | 3-$CF_3$ | H | - | 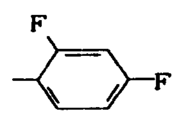 | | 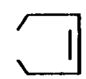 | 115 |

Tabelle 2 (Fortsetzung)

| Bsp.Nr. | X | Y | $Z_n$ | A | C | D | Schmelzpunkt/°C |
|---|---|---|---|---|---|---|---|
| 126 | H | H | - | 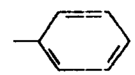 | H | $CH_3$ | 178 |
| 127 | $3-CF_3$ | H | - | 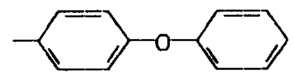 | H | $CH_3$ | Öl |
| 128 | 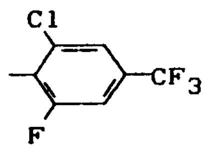 | H | - | 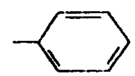 | H | $CH_3$ | 190 |
| 129 | 2-F | 4-F | - | 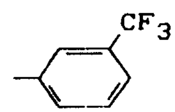 | H | $CH_3$ | 168 |
| 130 | $3-CF_3$ | H | - | 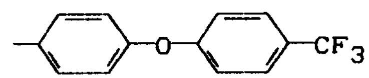 | H | $CH_3$ | Öl |

Tabelle 2 (Fortsetzung)

| Bsp.Nr. | X | Y | $Z_n$ | A | C | D | Schmelzpunkt/° C |
|---|---|---|---|---|---|---|---|
| 131 | H | H | - | 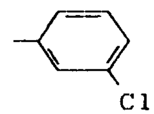 | H | $CH_3$ | 157 |
| 132 | H | H | - | 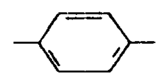 | H | $CH_3$ | 188 |
| 133 | H | H | - | 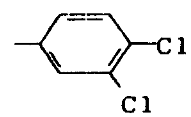 | H | $CH_3$ | 152 |
| 134 | 3-O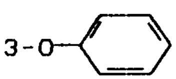 | H | - | 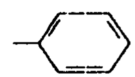 | H | $CH_3$ | 203 |
| 135 | 3-O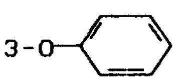 | H | - | 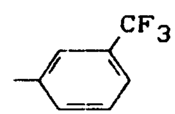 | H | $CH_3$ | 166 |
| 136 | 3-$CF_3$ | H | - | $CH_3$ | H | H | 167 |

<u>Tabelle 2</u> (Fortsetzung)

| Bsp.Nr. | X | Y | $Z_n$ | A | C | D | Schmelzpunkt/°C |
|---|---|---|---|---|---|---|---|
| 137 | 3-$CF_3$ | H | - | 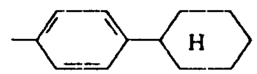 | H | $CH_3$ | 142 |
| 138 | 2-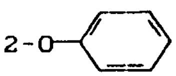 | H | - | 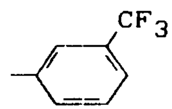 | H | $CH_3$ | 160 |
| 139 | H | H | - | 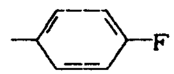 | H | $CH_3$ | 196 |
| 140 | 3-$CF_3$ | H | - | 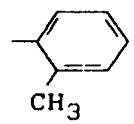 | H | $CH_3$ | 128 |
| 141 | 3-$CF_3$ | H | - | 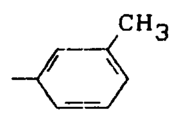 | H | $CH_3$ , | 67 |
| 142 | 3-$CF_3$ | H | - | 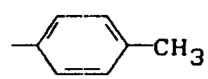 | H | $CH_3$ | 194 |

Tabelle 2 (Fortsetzung)

| Bsp.Nr. | X | Y | $Z_n$ | A | C | D | Schmelzpunkt/°C |
|---|---|---|---|---|---|---|---|
| 143 | 4-$CH_3$ | H | - | 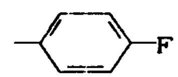 | H | $CH_3$ | 189 |
| 144 | 2-$CH_3$ | H | - | 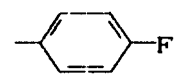 | H | $CH_3$ | 246-247 |
| 145 | 2-$CH_3$ | H | - | 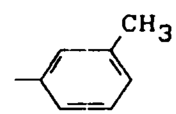 | H | $CH_3$ | 152 |
| 146 | 3-F | H | - | 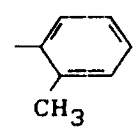 | H | $CH_3$ | 190 |
| 147 | 4-F | H | - | 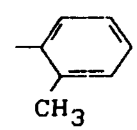 | H | $CH_3$ | 131 |
| 148 | 2-F | H | - | 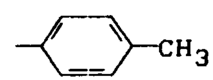 | H | $CH_3$ | 242-243 |

Tabelle 2 (Fortsetzung)

| Bsp.Nr. | X | Y | $Z_n$ | A | C | D | Schmelz-punkt/°C |
|---|---|---|---|---|---|---|---|
| 149 | 2-F | H | - | 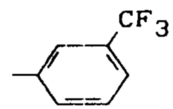 | H | $CH_3$ | 139 |
| 150 | 4-F | H | - | 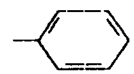 | H | $CH_3$ | 146 |
| 151 | 2,4-$F_2$ | H | - | 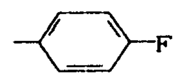 | H | $CH_3$ | 198 |
| 152 | 2,4-$F_2$ | H | - | 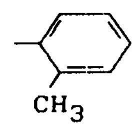 | H | $CH_3$ | 184 |
| 153 | 2,4-$F_2$ | H | - | 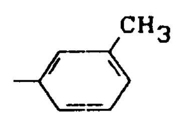 | H | $CH_3$ . | 214-215 |
| 154 | 2,4-$F_2$ | H | - | 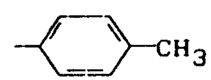 | H | $CH_3$ | 230-231 |
| 155 | 2-$CH_3$ | H | - | 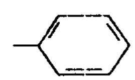 | H | $CH_3$ | 234 |

Beispiel 156

Verfahren (b)

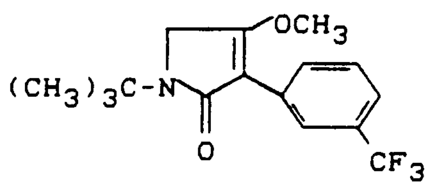

3-(3-Trifluormethylphenyl)-1-tert.butyl-4-methoxy-3-pyrrolin-2-on

3,89 g (13 mMol) 3-(3-Trifluormethylphenyl)-1-tert.-butyl-pyrrolidin-2,4-dion werden in 30 ml absolutem Acetonitril suspendiert und nach Zugabe von 0,84 g (15 mMol) pulverisiertem Kaliumhydroxid und 2,8 g (15 mMol) p-Toluolsulfonsäure-methylester gelöst in 5 ml absolutem Acetonitril 3 Stunden rückflussiert. Man rührt in 200 ml Wasser ein, extrahiert mit Methylenchlorid, trocknet und engt ein. Den Rückstand reinigt man durch Säulenchromatographie an Kieselgel mit Cyclohexan/Essigester 1:2.

Nach Kristallisation aus Ether/n-Hexan erhält man 2,03 g (47,2 % der Theorie) 3-(3-Trifluormethylphenyl)-1-tert.-butyl-4-methoxy-3-pyrrolin-2-on vom Schmelzpunkt 129 °C.

Analog erhält man:

Beispiel 157

3-(3-Trifluormethylphenyl)-1-(2-methoxyethyl)-4-methoxy-3-pyrrolin-2-on in einer Ausbeute von 56,2 % als Öl.

Anwendungsbeispiele

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen die Verbindungen 82, 89 und 92 eine sehr gute herbizide Wirkung gegen dikotyle Unkräuter bei einer sehr guten Nutzpflanzenverträglichkeit, wie beispielsweise in Baumwolle, Weizen oder Gerste.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen die Verbindungen 81, 88, 89 und 92 eine sehr gute herbizide Wirkung gegen mono- und dikotyle Unkräuter bei ausgezeichneter Nutzpflanzenverträglichkeit, wie beispielsweise in Gerste, Mais, Soja oder Baumwolle.

## Patentansprüche

**1.** 4-Alkoxy- bzw. 4-(substituierte) Amino-3-arylpyrrolinon-Derivate der allgemeinen Formel (I),

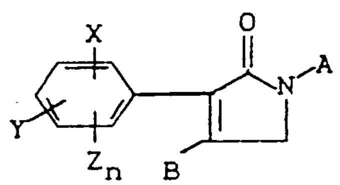

(I)

in welcher

X und Y unabhängig voneinander für Wasserstoff, Fluor, Chlor, Brom, Iod, $C_1$-$C_6$-Alkyl, $C_1$-$C_6$-Alkoxy, Halogen-$C_1$-$C_4$-alkyl, jeweils unsubstituiertes oder einfach bis fünffach, gleich oder verschieden substituiertes Phenoxy oder Phenylthio stehen, wobei als Phenylsubstituenten ausgewählt sind: Fluor, Chlor, Brom, Iod, $C_1$-$C_4$-Alkyl, $C_1$-$C_4$-Alkoxy und Halogen-$C_1$-$C_4$-alkyl,

Z für Fluor, Chlor, Brom, Iod, $C_1$-$C_6$-Alkyl oder $C_1$-$C_6$-Alkoxy steht,

n für eine Zahl 0, 1, 2 oder 3 steht,

A für jeweils unsubstituiertes oder jeweils durch Halogen substituiertes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_1$-$C_8$-alkyl, $C_1$-$C_{10}$-Alkylthio-$C_1$-$C_8$-alkyl oder für Cycloalkyl mit 3 bis 8 Ringatomen steht, das durch Sauerstoff, Stickstoff und/oder Schwefel unterbrochen sein kann, oder A für jeweils unsubstituiertes oder im Phenylteil einfach bis fünffach, gleich oder verschieden substituiertes Phenyl-$C_1$-$C_6$-alkyl, Phenyl oder Naphthyl steht, wobei als Substituenten jeweils ausgewählt sind: Fluor, Chlor, Brom, Iod, $C_1$-$C_6$-Alkyl, $C_1$-$C_6$-Alkoxy, Halogen-$C_1$-$C_6$-alkyl, Halogen-$C_1$-$C_6$-alkoxy oder jeweils unsubstituiertes oder einfach bis fünffach, gleich oder verschieden durch Fluor, Chlor, Brom, Iod, $C_1$-$C_4$-Alkyl, $C_1$-$C_4$-Alkoxy, Halogen-$C_1$-$C_4$-alkyl und Halogen-$C_1$-$C_4$-alkoxy substituiertes Phenoxy oder Phenylthio,

B für Hydroxy, $C_1$-$C_{10}$-Alkoxy, Halogen-$C_1$-$C_{10}$-alkoxy oder Cycloalkoxy mit 3 bis 7 Ringatomen steht, wobei der Cycloalkyl-Rest unsubstituiert oder einfach bis fünffach, gleich oder verschieden durch Fluor, Chlor, Brom und $C_1$-$C_4$-Alkyl substituiert ist, oder

B für die Gruppe

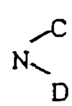

steht, wobei

C und D unabhängig voneinander für Wasserstoff, $C_1$--$C_{10}$-Alkyl, $C_3$-$C_{10}$-Alkenyl, $C_1$-$C_8$-Alkoxy-$C_1$-$C_8$-alkyl, $C_1$-$C_8$-Alkylthio-$C_1$-$C_8$-alkyl, $C_1$-$C_8$-Polyalkoxy-$C_1$-$C_8$-alkyl, Cycloalkyl mit 3 bis 8 Ringatomen, das durch ein, zwei oder drei gleiche oder verschiedene Sauerstoff-,

Stickstoff- und Schwefelatome unterbrochen sein kann, jeweils unsubstituiertes oder einfach bis fünffach, gleich oder verschieden substituiertes Phenyl, Phenyl-$C_1$-$C_6$-alkyl, Phenoxy-$C_1$-$C_6$-alkyl, Hetaryl oder Hetaryl-$C_1$-$C_6$-alkyl mit jeweils 5 oder 6 Ringatomen, wobei 1 oder 2 gleiche oder verschiedene Sauerstoff-, Stickstoff- und Schwefelatome im Ring enthalten sind, oder

C und D zusammen mit dem Stickstoff, an den sie gebunden sind, einen 3- bis 8-gliedrigen Ring bilden, der ein oder zwei Doppelbindungen enthalten kann, der ein oder zwei weitere, gleiche oder verschiedene Sauerstoff-, Schwefel- oder Stickstoffatome enthalten kann, und der einfach bis fünffach, gleich oder verschieden durch $C_1$-$C_4$-Alkyl und $C_1$-$C_4$-Alkylcarbonyl substituiert sein kann,

mit der Maßgabe, daß wenigstens ein Rest X, Y oder Z nicht für Chlor oder Methyl steht, wenn B für Hydroxy steht und A eine andere Bedeutung als gegebenenfalls substituiertes Phenyl oder Naphthyl hat und wenigstens ein Rest X, Y oder Z eine andere Bedeutung als Wasserstoff hat, wenn B für Hydroxy steht und A die Bedeutung von 2,6-Diethylphenyl hat.

**2.** 4-Alkoxy- bzw. 4(substituierte)Amino-3-arylpyrrolinon-Derivate der Formel (I) gemäß Anspruch 1, in welcher

B für Hydroxy, Methoxy, Ethoxy, n- oder iso-Propoxy, n-, iso-, sec.- oder tert.-Butoxy, Halogen-$C_1$-$C_4$-alkoxy mit Fluor und/oder Chloratomen oder Cycloalkoxy mit 3 bis 6 Ringatomen steht, wobei der Cycloalkyl-Rest unsubstituiert oder einfach bis dreifach, gleich oder verschieden durch Fluor, Chlor, Methyl und Ethyl substituiert ist,

X für Wasserstoff, Fluor, Chlor, Methyl, Ethyl, n-oder iso-Propyl, Methoxy, Ethoxy, n- oder i-Propoxy, Halogenmethyl mit 1, 2 oder 3 Fluor- und/oder Chloratomen, Halogenethyl mit 1 bis 5 Fluor- und/oder Chloratomen, jeweils unsubstituiertes oder einfach bis fünffach gleich oder verschieden substituiertes Phenoxy oder Phenylthio, wobei als Phenylsubstituenten ausgewählt sind: Fluor, Chlor, Brom, Methyl, Ethyl, n- oder iso-Propyl, Methoxy, Ethoxy, n- oder iso-Propoxy und Trifluormethyl,

Y für Wasserstoff, Fluor, Chlor, Methyl, Ethyl, Methoxy, Ethoxy oder Trifluormethyl steht,

Z für Fluor oder Chlor steht,

n für 0, 1, 2 oder 3 steht und

A für Methyl, Ethyl, jeweils geradkettiges oder verzweigtes Propyl, Butyl, Pentyl, Hexyl, Heptyl, Octyl, Nonyl oder Decyl, Halogen-$C_1$-$C_6$-alkyl,$C_3$-$C_6$-Alkenyl, $C_3$-$C_6$-Alkinyl, $C_1$-$C_4$-Alkoxy-$C_1$-$C_4$-alkyl, $C_1$-$C_4$-Alkylthio-$C_1$-$C_4$-alkyl steht oder für Cycloalkyl mit 3 bis 6 Ringatomen steht, das durch Sauerstoff, Stickstoff oder Schwefel unterbrochen sein kann, oder A für jeweils unsubstituiertes oder einfach bis fünffach, insbesondere einfach bis dreifach, gleich oder verschieden substituiertes Phenyl, Benzyl oder Phenethyl steht, wobei als Substituenten jeweils ausgewählt sind: Fluor, Chlor, Methyl, Ethyl, n-oder iso-Propyl, Methoxy, Ethoxy, Trifluormethyl, Trifluormethoxy oder jeweils unsubstituiertes oder einfach bis dreifach, gleich oder verschieden, durch Fluor, Chlor, Methyl, Methoxy, Trifluormethyl und Trifluormethoxy substituiertes Phenoxy oder Phenylthio,

mit der Maßgabe, daß wenigstens ein Rest X, Y oder Z nicht für Chlor oder Methyl steht, wenn B für Hydroxy und A eine andere Bedeutung als gegebenenfalls substituiertes Phenyl hat und wenigstens ein Rest X, Y oder Z eine andere Bedeutung als Wasserstoff hat, wenn B für Hydroxy steht und A die Bedeutung von 2,6-Diethylphenyl hat.

**3.** 4-Alkoxy- bzw. 4(substituierte)Amino-3-arylpyrrolinon-Derivate der Formel (I) gemäß Anspruch 1, in welcher

B für die Gruppe

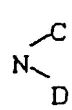

steht, wobei

C für Wasserstoff, Methyl, Ethyl, n- oder iso-Propyl steht und

D für Methyl, Ethyl, n- oder iso-Propyl, n-, iso-, sec.- oder tert.-Butyl, Allyl, Propargyl, Methoxymethyl, Methoxyethyl, Ethoxymethyl, Ethoxyethyl, Methylthiomethyl, Methylthioethyl, Ethylthiomethyl, Ethylthioethyl, Cyclopropyl, Cyclopentyl, Cyclohexyl, jeweils unsubstituiertes oder einfach bis fünffach, insbesondere einfach bis dreifach, gleich oder verschieden durch Fluor, Chlor, Methyl, Ethyl und Trifluormethyl substituiertes Phenyl, Benzyl oder Phenethyl, für Pyridyl oder Pyrimidyl steht oder

C und D zusammen mit dem Stickstoff, an den sie gebunden sind, einen 5- oder 6-gliedrigen Ring bilden, der ein oder zwei Doppelbindungen enthalten kann oder der ein weiteres Stickstoff-, Sauerstoff- oder Schwefelatom enthalten kann und der einfach bis dreifach, gleich oder verschieden durch Methyl, Ethyl, Methylcarbonyl und Ethylcarbonyl substituiert sein kann,

X für Wasserstoff, Fluor, Chlor, Methyl, Ethyl, n-oder iso-Propyl, Methoxy, Ethoxy, n- oder i-Propoxy, Halogenmethyl mit 1, 2 oder 3 Fluor- und/oder Chloratomen, Halogenethyl mit 1 bis 5 Fluor- und/oder Chloratomen, jeweils unsubstituiertes oder einfach bis fünffach, gleich oder verschieden substituiertes Phenoxy oder Phenylthio, wobei als Phenylsubstituenten ausgewählt sind: Fluor, Chlor, Brom, Methyl, Ethyl, n- oder iso-Propyl, Methoxy, Ethoxy, n- oder iso-Propoxy und Trifluormethyl,

Y für Wasserstoff, Fluor, Chlor, Methyl, Ethyl, Methoxy, Ethoxy oder Trifluormethyl steht,

Z für Fluor oder Chlor steht,

n für 0, 1, 2 oder 3 steht und

A für Methyl, Ethyl, jeweils geradkettiges oder verzweigtes Propyl, Butyl, Pentyl, Hexyl, Heptyl, Octyl, Nonyl oder Decyl, Halogen-$C_1$-$C_6$-alkyl,$C_3$-$C_6$-Alkenyl, $C_3$-$C_6$-Alkinyl, $C_1$-$C_4$-Alkoxy-$C_1$-$C_4$-alkyl, $C_1$-$C_4$-Alkylthio-$C_1$-$C_4$-alkyl steht oder für Cycloalkyl mit 3 bis 6 Ringatomen steht, das durch Sauerstoff, Stickstoff oder Schwefel unterbrochen sein kann, oder A für jeweils unsubstituiertes oder einfach bis fünffach, insbesondere einfach bis dreifach, gleich oder verschieden substituiertes Phenyl, Benzyl oder Phenethyl steht, wobei als Substituenten jeweils ausgewählt sind: Fluor, Chlor, Methyl, Ethyl, n- oder iso-Propyl, Methoxy, Ethoxy, Trifluormethyl, Trifluormethoxy oder jeweils unsubstituiertes oder einfach bis dreifach, gleich oder verschieden, durch Fluor, Chlor, Methyl, Methoxy, Trifluormethyl und Trifluormethoxy substituiertes Phenoxy oder Phenylthio.

**4.** Verfahren zur Herstellung von 4-Alkoxy- bzw. 4-(substituierte) Amino-3-arylpyrrolinon-Derivaten der allgemeinen Formel (I)

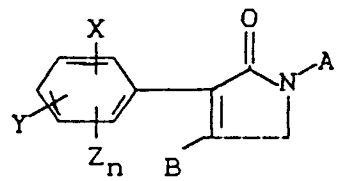

(I)

in welcher

X, Y, Z, n, A und B die in Anspruch 1 angegebene Bedeutung haben

dadurch gekennzeichnet, daß man

a) Verbindungen der Formel (I), in denen B für Hydroxyl und in welcher die Reste A, X, Y und $Z_n$ die oben angegebene Bedeutung haben, erhält, wenn man

N-Acylaminosäureester der Formel (II)

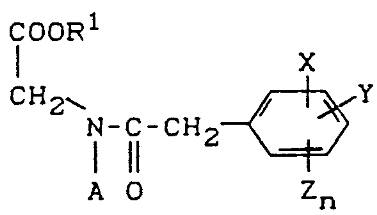

(II)

in welcher

A, X, Y, Z und n die oben angegebene Bedeutung haben, und

$R^1$ für Alkyl, insbesondere Methyl oder Ethyl, steht,
in Gegenwart eines Verdünnungsmittels und in Gegenwart einer Base intramolekular kondensiert,
oder daß man
b) Verbindungen der Formel (I), in denen
B für Alkoxy, Halogenalkoxy oder Cycloalkoxy steht, wobei der Cycloalkyl-Rest unsubstituiert oder durch Halogen und/oder Alkyl substituiert ist, und
A, X, Y, Z und n die oben angegebene Bedeutung haben,
erhält, wenn man Verbindungen der Formel (I), in denen B für Hydroxyl steht,
mit Alkylierungsreagenzien der Formel (III)

$R^2$ - $X^1$ (III)

in welcher
$R^2$ für Alkyl, Halogenalkyl oder Cycloalkyl steht, wobei der Cycloalkyl-Rest unsubstituiert oder durch Halogen und/oder Alkyl substituiert ist, und
$X^1$ für Halogen oder eine andere für Alkylierungsreagenzien übliche Abgangsgruppe steht,
gegebenenfalls in Gegenwart eines Verdünnungsmittels und gegebenenfalls in Gegenwart eines Säurebindemittels umsetzt,
oder daß man
c) Verbindungen der Formel (I), in denen
B für

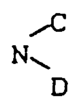

steht und
A, C, D, X, Y, Z und n die oben angegebene Bedeutung haben,
erhält, wenn man Verbindungen der Formel (I), in denen B für Hydroxyl steht,
mit Aminen der Formel (IV)

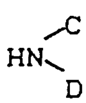
(IV)

in welcher
C und D die oben angegebene Bedeutung haben,
gegebenenfalls in Gegenwart eines Verdünnungsmittels und gegebenenfalls in Gegenwart eines wasserentziehenden Mittels umsetzt.

**5.** Herbizide Mittel, gekennzeichnet durch einen Gehalt an mindestens einem 4-Alkoxy- bzw. 4-(substituierten)Amino-3-arylpyrrolinon-Derivat der Formel (I) gemäß den Ansprüchen 1 bis 4.

**6.** Verfahren zur Bekämpfung von unerwünschten Pflanzen, dadurch gekennzeichnet, daß man 4-Alkoxy- bzw. 4-(substituierten)Amino-3-aryl-pyrrolinon-Derivate der Formel (I) gemäß den Ansprüchen 1 bis 4 auf die Pflanzen und/oder ihren Lebensraum einwirken läßt.

**7.** Verwendung von 4-Alkoxy- bzw. 4-(substituierte)Amino-3-arylpyrrolinon-Derivate der Formel (I) gemäß den Ansprüchen 1 bis 4 zur Bekämpfung von unerwünschten Pflanzen.

**8.** Verfahren zur Herstellung von herbiziden Mitteln, dadurch gekennzeichnet, daß man 4-Alkoxy- bzw. 4-(substituierte)Amino-3-arylpyrrolinon-Derivate der Formel (I) gemäß den Ansprüchen 1 bis 4 mit Streckmitteln und/oder oberflächenaktiven Substanzen vermischt.

## Claims

**1.** 4-Alkoxy- and 4-(substituted) amino-3-arylpyrrolinone derivatives of the general formula (I),

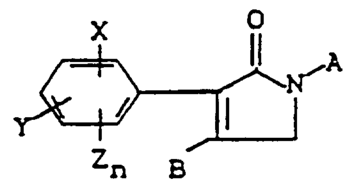

(I)

in which

X and Y independently of one another represent hydrogen, fluorine, chlorine, bromine, iodine, $C_{1-6}$-alkyl, $C_1$-$C_6$-alkoxy, halogeno-$C_1$-$C_4$-alkyl or phenoxy or phenylthio which are in each case unsubstituted or substituted by one to five identical or different substituents, the substituents selected for the phenyl being: fluorine, chlorine, bromine, iodine, $C_1$-$C_4$-alkyl, $C_1$-$C_4$-alkoxy and halogeno-$C_1$-$C_4$-alkyl,

Z represents fluorine, chlorine, bromine, iodine, $C_1$-$C_6$-alkyl or $C_1$-$C_6$-alkoxy,

n represents the number 0, 1, 2 or 3,

A represents in each case unsubstituted or in each case halogen-substituted $C_1$-$C_{12}$-alkyl, $C_3$-$C_8$-alkenyl, $C_3$-$C_8$-alkinyl, $C_1$-$C_{10}$-alkoxy-$C_2$-$C_8$-alkyl, $C_1$-$C_8$-polyalkoxy-$C_1$-$C_8$-alkyl or $C_1$-$C_{10}$-alkylthio-$C_1$-$C_8$-alkyl, or represents cycloalkyl which has 3 to 8 ring atoms and can be interrupted by oxygen, nitrogen and/or sulphur, or A represents phenyl-$C_1$-$C_6$-alkyl, phenyl or naphthyl, in each case unsubstituted or substituted in the phenyl part by one to five identical or different substituents, the substituents selected in each case being: fluorine, chlorine, bromine, iodine, $C_1$-$C_6$-alkyl, $C_1$-$C_6$-alkoxy, halogeno-$C_1$-$C_6$-alkyl, halogeno-$C_1$-$C_6$-alkoxy or phenoxy or phenylthio which are in each case unsubstituted or substituted by one to five identical or different substituents from the group comprising fluorine, chlorine, bromine, iodine, $C_1$-$C_4$-alkyl, $C_1$-$C_4$-alkoxy, halogeno-$C_1$-$C_4$-alkyl and halogeno-$C_1$-$C_4$-alkoxy,

B represents hydroxyl, $C_1$-$C_{10}$-alkoxy, halogeno-$C_1$-$C_{10}$-alkoxy or cycloalkoxy having 3 to 7 ring atoms, the cycloalkyl radical being unsubstituted or substituted by one to five identical or different substituents from the group comprising fluorine, chlorine, bromine and $C_1$-$C_4$-alkyl, or

B represents the group

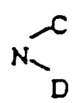

,

wherein

C and D independently of one another represent hydrogen, $C_1$-$C_{10}$-alkyl, $C_3$-$C_{10}$-alkenyl, $C_1$-$C_8$-alkoxy-$C_1$-$C_8$-alkyl, $C_1$-$C_8$-alkylthio-$C_1$-$C_8$-alkyl, $C_1$-$C_8$-polyalkoxy-$C_1$-$C_8$-alkyl, cycloalkyl having 3 to 8 ring atoms, which can be interrupted by one, two or three identical or different oxygen, nitrogen and sulphur atoms, or phenyl, phenyl-$C_1$-$C_6$-alkyl, phenoxy-$C_1$-$C_6$-alkyl, hetaryl or hetaryl-$C_1$-$C_6$-alkyl, having in each case 5 or 6 ring atoms and containing 1 or 2 identical or different oxygen, nitrogen and sulphur atoms in the ring, which are in each case unsubstituted or substituted by one to five identical or different substituents, or

C and D, together with the nitrogen to which they are bonded, form a 3- to 8- membered ring which can contain one or two double bonds, can contain one or two further identical or different oxygen, sulphur or nitrogen atoms and can be substituted by one to five identical or different substituents from the group comprising $C_1$-$C_4$-alkyl and $C_1$-$C_4$-alkylcarbonyl,

with the proviso that at least one radical X, Y or Z does not represent chlorine or methyl if B represents hydroxyl and A has a meaning other than optionally substituted phenyl or naphthyl and at least one

radical X, Y or Z has a meaning other than hydrogen if B represents hydroxyl and A has the meaning of 2,6-diethylphenyl.

**2.** 4-Alkoxy- and 4-(substituted) amino-3-arylpyrrolinone derivatives of the formula (I) according to Claim 1, in which

B represents hydroxyl, methoxy, ethoxy, n- or iso-propoxy, n-, iso-, sec. - or tert.-butoxy, halogeno-$C_1$-$C_4$-alkoxy, containing fluorine and/or chlorine atoms, or cycloalkoxy having 3 to 6 ring atoms, the cycloalkyl radical being unsubstituted or substituted by one to three identical or different substituents from the group comprising fluorine, chlorine, methyl and ethyl,

X represents hydrogen, fluorine, chlorine, methyl, ethyl, n- or iso-propyl, methoxy, ethoxy, n- or i-propoxy, halogenomethyl containing 1, 2 or 3 fluorine and/or chlorine atoms, halogenoethyl containing 1 to 5 fluorine and/or chlorine atoms, or phenoxy or phenylthio which are in each case unsubstituted or substituted by one to five identical or different substituents, the substituents selected for the phenyl being: fluorine, chlorine, bromine, methyl, ethyl, n- or iso-propyl, methoxy, ethoxy, n- or iso-propoxy and trifluoromethyl,

Y represents hydrogen, fluorine, chlorine, methyl, ethyl, methoxy, ethoxy or trifluoromethyl,

Z represents fluorine or chlorine,

n represents 0, 1, 2 or 3 and

A represents methyl, ethyl, in each case straight-chain or branched propyl, butyl, pentyl, hexyl, heptyl, octyl, nonyl or decyl, halogeno-$C_1$-$C_6$-alkyl, $C_3$-$C_6$-alkenyl, $C_3$-$C_6$-alkinyl, $C_1$-$C_4$-alkoxy-$C_1$-$C_4$-alkyl or $C_1$-$C_4$-alkylthio-$C_1$-$C_4$-alkyl, or represents cycloalkyl which has 3 to 6 ring atoms and can be interrupted by oxygen, nitrogen or sulphur, or A represents phenyl, benzyl or phenethyl which are in each case unsubstituted or substituted by one to five, in particular one to three, identical or different substituents, the substituents selected in each case being: fluorine, chlorine, methyl, ethyl, n- or iso-propyl, methoxy, ethoxy, trifluoromethyl, trifluoromethoxy or phenoxy or phenylthio, which are in each case unsubstituted or substituted by one to three identical or different substituents from the group comprising fluorine, chlorine, methyl, methoxy, trifluoromethyl and trifluoromethoxy,

with the proviso that at least one radical X, Y or Z does not represent chlorine or methyl if B represents hydroxyl and A has a meaning other than optionally substituted phenyl and at least one radical X, Y or Z has a meaning other than hydrogen if B represents hydroxyl and A has the meaning of 2,6-diethylphenyl.

**3.** 4-Alkoxy- and 4-(substituted) amino-3-arylpyrrolinone derivatives of the formula (I) according to Claim 1, in which

B represents the group

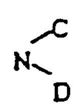

,

wherein

C represents hydrogen, methyl, ethyl or n- or iso-propyl and

D represents methyl, ethyl, n- or iso-propyl, n-, iso-, sec. - or tert.-butyl, allyl, propargyl, methoxymethyl, methoxyethyl, ethoxymethyl, ethoxyethyl, methylthiomethyl, methylthioethyl, ethylthiomethyl, ethylthioethyl, cyclopropyl, cyclopentyl, cyclohexyl, or phenyl, benzyl or phenethyl, which are in each case unsubstituted or substituted by one to five, in particular one to three, identical or different substituents from the group comprising fluorine, chlorine, methyl, ethyl and trifluoromethyl, or represents pyridyl or pyrimidyl, or

C and D , together with the nitrogen to which they are bonded, form a 5- or 6-membered ring which can contain one or two duble bonds or can contain a further nitrogen, oxygen or sulphur atom and can be substituted by one to three identical or different substituents from the group comprising methyl, ethyl, methylcarbonyl and ethylcarbonyl,

X represents hydrogen, fluorine, chlorine, methyl, ethyl, n- or iso-propyl, methoxy, ethoxy, n- or i-propoxy, halogenomethyl which contains 1, 2 or 3 fluorine and/or chlorine atoms, halogenoethyl which contains 1 to 5 fluorine and/or chlorine atoms, or phenoxy or phenylthio which are in each case unsubstituted or substituted by one to five identical

or different substituents, the substituents selected for the phenyl being: fluorine, chlorine, bromine, methyl, ethyl, n- or iso-propyl, methoxy, ethoxy, n- or iso-propoxy and trifluoromethyl,

Y represents hydrogen, fluorine, chlorine, methyl, ethyl, methoxy, ethoxy or trifluoromethyl,

Z represents fluorine or chlorine,

n represents 0, 1, 2 or 3 and

A represents methyl, ethyl, in each case straight-chain or branched propyl, butyl, pentyl, hexyl, heptyl, octyl, nonyl or decyl, halogeno-$C_1$-$C_6$-alkyl, $C_3$-$C_6$-alkenyl, $C_3$-$C_6$-alkinyl, $C_1$-$C_4$-alkoxy-$C_1$-$C_4$-alkyl or $C_1$-$C_4$-alkylthio-$C_1$-$C_4$-alkyl, or represents cycloalkyl which has 3 to 6 ring atoms and can be interrupted by oxygen, nitrogen or sulphur, or A represents phenyl, benzyl or phenethyl, in each case unsubstituted or substituted by one to five, in particular one to three, identical or different substituents, the substituents selected in each case being: fluorine, chlorine, methyl, ethyl, n- or iso-propyl, methoxy, ethoxy, trifluoromethyl, trifluoromethoxy or phenoxy or phenylthio, which are in each case unsubstituted or substituted by one to three identical or different substituents from the group comprising fluorine, chlorine, methyl, methoxy, trifluoromethyl and trifluoromethoxy.

**4.** Process for the preparation of 4-alkoxy- and 4-(substituted) amino-3-arylpyrrolinone derivatives of the general formula (I)

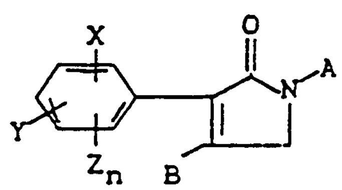

(I)

in which

X, Y, Z, n, A and B have the meaning given in Claim 1, characterized in that

a) compounds of the formula (I) in which B represents hydroxyl and in which the radicals A, X, Y and $Z_n$ have the abovementioned meaning are obtained by a process in which N-acylamino acid esters of the formula (II)

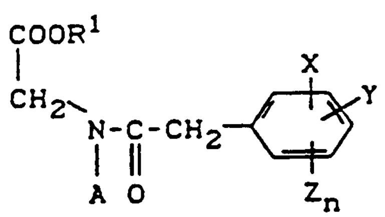

(II)

in which

A, X, Y, Z and n have the abovementioned meaning and

$R^1$ represents alkyl, in particular methyl or ethyl,

are subjected to intramolecular condensation in the presence of a diluent and in the presence of a base, or in that

b) compounds of the formula (I) in which

B represents alkoxy, halogenoalkoxy or cycloalkoxy, the cycloalkyl radical being unsubstituted or substituted by halogen and/or alkyl, and

A, X, Y, Z and n have the abovementioned meaning,

are obtained by a process in which compounds of the formula (I) in which B represents hydroxyl are reacted with alkylating reagents of the formula (III)

$R^2$ - $X^1$ (III)

in which

$R^2$ represents alkyl, halogenoalkyl or cycloalkyl, the cycloalkyl radical being unsubstituted or substituted by halogen and/or alkyl, and

$X^1$ represents halogen or another leaving group customary for alkylating reagents,

if appropriate in the presence of a diluent and if appropriate in the presence of an acid-binding agent,

or in that

c) compounds of the formula (I) in which

B represents

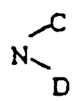

and

A, C, D, X, Y, Z and n have the abovementioned meaning,

are obtained by a process in which compounds of the formula (I) in which B represents hydroxyl are reacted with amines of the formula (IV)

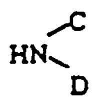

(IV)

in which

C and D have the abovementioned meaning,

if appropriate in the presence of a diluent and if appropriate in the presence of a dehydrating agent.

5. Herbicidal agents, characterized in that they contain at least one 4-alkoxy- or 4-(substituted) amino-3-arylpyrrolinone derivative of the formula (I) according to Claims 1 to 4.

6. Method of combating undesirable plants, characterized in that 4-alkoxy- or 4-(substituted) amino-3-arylpyrrolinone derivatives of the formula (I) according to Claims 1 to 4 are allowed to act on the plants and/or their environment.

7. Use of 4-alkoxy- and 4-(substituted) amino-3-arylpyrrolinone derivatives of the formula (I) according to Claims 1 to 4 for combating undesirable plants.

8. Process for the preparation of herbicidal agents, characterized in that 4-alkoxy- and 4-(substituted) amino-3-arylpyrrolinone derivatives of the formula (I) according to Claims 1 to 4 are mixed with extenders and/or surface-active substances.

**Revendications**

1. Dérivés de 4-alkoxy- et 4-(amino substitué)-3-aryl-pyrrolidones de formule générale (I)

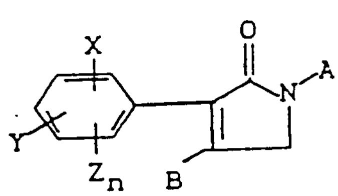

(I)

dans laquelle

X et Y représentent, indépendamment l'un de l'autre, de l'hydrogène, du fluor, du chlore, du brome, de l'iode, un groupe alkyle en $C_1$ à $C_6$, alkoxy en $C_1$ à $C_6$, halogénalkyle en $C_1$ à $C_4$, un groupe phénoxy ou un groupe phénylthio non substitués ou portant chacun un à cinq substituants égaux ou différents, en choisissant comme substituants du groupe phényle : le fluor, le chlore, le brome, l'iode, un radical alkyle en $C_1$ à $C_4$, alkoxy en $C_1$ à $C_4$ et halogénalkyle en $C_1$ à $C_4$,

Z est du fluor, du chlore, du brome, de l'iode, un groupe alkyle en $C_1$ à $C_6$ ou alkoxy en $C_1$ à $C_6$,

n représente le nombre 0, 1, 2 ou 3,

A désigne des groupes alkyle en $C_1$ à $C_{12}$, alcényle en $C_3$ à $C_8$, alcynyle en $C_3$ à $C_8$ -(alkoxy en $C_1$ à $C_{10}$)-(alkyle en $C_2$ à $C_8$), poly(alkoxy en $C_1$ à $C_8$)-(alkyle en $C_1$ à $C_8$), (alkylthio en $C_1$ à $C_{10}$)-(alkyle en $C_1$ à $C_8$) ne portant pas de substituant ou substitués chacun par un halogène, ou un groupe cycloalkyle à noyau de 3 à 8 atomes, qui peut être interrompu par de l'oxygène, de l'azote et/ou du soufre, ou bien A représente un groupe phényl-(alkyle en $C_1$ à $C_6$), phényle ou naphtyle ne portant pas de substituant ou portant chacun dans la partie phényle un à cinq substituants égaux ou différents, en choisissant dans chaque cas comme substituants : le fluor, le chlore, le brome, l'iode, un radical alkyle en $C_1$ à $C_6$, alkoxy en $C_1$ à $C_6$, halogénalkyle en $C_1$ à $C_6$, halogénalkoxy en $C_1$ à $C_6$, ou un groupe phénoxy ou phénylthio ne portant pas de substituant ou portant chacun un à cinq substituants, égaux ou différents, fluoro, chloro, bromo, iodo, alkyle en $C_1$ à $C_4$, alkoxy en $C_1$ à $C_4$, halogénalkyle en $C_1$ à $C_4$ et halogénalkoxy en $C_1$ à $C_4$,

B est un groupe hydroxy, alkoxy en $C_1$ à $C_{10}$, halogénalkoxy en $C_1$ à $C_{10}$ ou cycloalkoxy à noyau de 3 à 7 atomes, le reste cycloalkyle ne portant pas de substituant ou étant substitué une à cinq fois, égales ou différentes, par du fluor, du chlore, du brome et un radical alkyle en $C_1$ à $C_4$, ou bien

B est le groupe

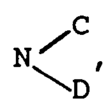

dans lequel

C et D représentent, indépendamment l'un de l'autre, de l'hydrogène, un groupe alkyle en $C_1$ à $C_{10}$, alcényle en $C_3$ à $C_{10}$, (alkoxy en $C_1$ à $C_8$)-(alkyle en $C_1$ à $C_8$), (alkylthio en $C_1$ à $C_8$)-(alkyle en $C_1$ à $C_8$), (polyalkoxy en $C_1$ à $C_8$)-(alkyle en $C_1$ à $C_8$), cycloalkyle à noyau de 3 à 8 atomes, qui peut être interrompu par 1, 2 ou 3 atomes d'oxygène, d'azote ou de soufre égaux ou différents, un groupe phényle, phényl(alkyle en $C_1$ à $C_6$), phénoxy(alkyle en $C_1$ à $C_6$), hétaryle ou hétaryl(alkyle en $C_1$ à $C_6$) à noyau de 5 ou 6 atomes, sans substituant ou portant chacun un à cinq substituants égaux ou différents, un ou deux atomes d'oxygène, d'azote et de soufre identiques ou différents étant contenus dans le noyau, ou bien

C et D forment conjointement avec l'azote auquel ils sont liés un noyau de 3 à 8 chaînons, qui peut contenir une ou deux doubles liaisons, qui peut comporter un ou deux autres atomes d'oxygène, de soufre ou d'azote identiques ou différents et qui peut être substitué une à cinq fois identiques ou différentes par un radical alkyle en $C_1$ à $C_4$ et (alkyle en $C_1$ à $C_4$)-carbonyle,

sous réserve qu'au moins un reste X, Y ou Z ne représente pas du chlore ou un groupe méthyle lorsque B est un groupe hydroxy et A a une définition autre qu'un groupe phényle ou naphtyle éventuellement substitué et qu'au moins un reste X, Y ou Z ait une définition autre qu'un atome d'hydrogène lorsque B est un groupe hydroxy et A est un groupe 2,6-diéthylphényle.

**2.** Dérivés de 4-alkoxy- et 4(amino substitué)-3-aryl-pyrrolidones de formule (I) suivant la revendication 1, dans laquelle :

B est un groupe hydroxy, méthoxy, éthoxy, n-propoxy, isopropoxy, n-butoxy, isobutoxy, sec.butoxy, tertio-butoxy, halogénalkoxy en $C_1$ à $C_4$ avec des atomes de fluor et/ou de chlore ou cycloalkoxy à noyau de 3 à 6 atomes, le reste cycloalkyle étant sans substituant ou portant 1 à 3 substituants fluoro, chloro, méthyle et éthyle égaux ou différents,

X représente de l'hydrogène, du fluor, du chlore, un groupe méthyle, éthyle, n-propyle, isopropyle, méthoxy, éthoxy, n-propoxy, isopropoxy, halogénométhyle ayant 1, 2 ou 3 atomes de fluor et/ou de chlore, halogénéthyle ayant 1 à 5 atomes de fluor et/ou de chlore, un groupe phénoxy ou phénylthio sans substituant ou portant chacun 1 à 5 substituants égaux ou différents, en choisissant comme substituants du groupe phényle : le fluor, le chlore, le brome, un radical méthyle, éthyle, n-propyle, isopropyle, méthoxy, éthoxy, n-propoxy, isopropoxy et trifluorométhyle,

Y est de l'hydrogène, du fluor, du chlore, un groupe méthyle, éthyle, méthoxy, éthoxy ou trifluorométhyle,

Z est du fluor ou du chlore,

n est la valeur 0, 1, 2 ou 3 et

A est un groupe méthyle, éthyle, un groupe propyle, butyle, pentyle, hexyle, heptyle, octyle, nonyle ou décyle linéaire ou ramifié dans chaque cas, un groupe halogénalkyle en $C_1$ à $C_6$, alcényle en $C_3$ à $C_6$, alcynyle en $C_3$ à $C_6$, (alkoxy en $C_1$ à $C_4$)-(alkyle en $C_1$ à $C_4$), (alkylthio en $C_1$ à $C_4$)-(alkyle en $C_1$ à $C_4$) ou un groupe cycloalkyle à noyau de 3 à 6 atomes, qui peut être interrompu par de l'oxygène, de l'azote ou du soufre, ou bien A représente un groupe phényle, benzyle ou phénéthyle, sans substituant ou portant chacun 1 à 5, notamment 1 à 3 substituants égaux ou différents, en choisissant dans chaque cas comme substituants : le fluor, le chlore, un radical méthyle, éthyle, n-propyle, isopropyle, méthoxy, éthoxy, trifluorométhyle, trifluorométhoxy, ou bien un groupe phénoxy ou phénylthio sans substituant ou portant chacun 1 à 3 substituants fluoro, chloro, méthyle, méthoxy, trifluorométhyle et trifluorométhoxy égaux ou différents,

sous réserve qu'au moins un reste X, Y ou Z ne représente pas du chlore ou un radical méthyle lorsque B est un groupe hydroxy et A a une définition autre qu'un groupe phényle éventuellement substitué, et qu'au moins un reste X, Y ou Z ait une définition autre que de l'hydrogène lorsque B représente un groupe hydroxy et A a la définition d'un reste 2,6-diéthylphényle.

**3.** Dérivés de 4-alkoxy- et 4-(amino substitué)-3-aryl-pyrrolidones de formule (I) suivant la revendication 1? dans laquelle :

B est le groupe

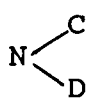,

où

C est de l'hydrogène, un radical méthyle, éthyle, n-propyle ou isopropyle, et

D est un groupe méthyle, éthyle, n-propyle, iso-propyle, n-butyle, iso-butyle, sec.butyle, tertio-butyle, allyle, propargyle, méthoxyméthyle, méthoxyéthyle, éthoxyméthyle, éthoxyéthyle, méthylthiométhyle, méthylthioéthyle, éthylthiométhyle, éthylthioéthyle, cyclopropyle, cyclopentyle, cyclohexyle, un groupe phényle, benzyle ou phénéthyle sans substituant ou portant chacun 1 à 5, notamment 1 à 3 substituants fluoro, chloro, méthyle, éthyle et trifluorométhyle égaux ou différents, un groupe pyridyle ou un groupe pyrimidyle, ou bien

C et D forment conjointement avec l'azote auquel ils sont liés un noyau pentagonal ou hexagonal qui peut contenir une ou deux doubles liaisons ou qui peut contenir un autre atome d'azote, d'oxygène ou de soufre et qui peut porter 1 à 3 substituants méthyle, éthyle, méthylcarbonyle et éthylcarbonyle égaux ou différents,

X représente de l'hydrogène, du fluor, du chlore, un groupe méthyle, éthyle, n-propyle, isopropyle, méthoxy, éthoxy, n-propoxy, isopropoxy, halogénométhyle ayant 1, 2 ou 3 atomes de fluor et/ou de chlore, halogénéthyle ayant 1 à 5 atomes de fluor et/ou de chlore, un groupe phénoxy ou phénylthio sans substituant ou portant chacun 1 à 5 substituants égaux ou différents, en choisissant comme substituants du groupe phényle : le fluor, le chlore, le brome, un radical méthyle, éthyle, n-propyle, isopropyle, méthoxy, éthoxy, n-propoxy, isopropoxy ou trifluorométhyle,

Y est de l'hydrogène, du fluor, du chlore, un groupe méthyle, éthyle, méthoxy, éthoxy ou trifluorométhyle,

Z est du fluor ou du chlore,

n a la valeur 0, 1, 2 ou 3 et

A est un groupe méthyle, éthyle, un groupe propyle, butyle, pentyle, hexyle, heptyle, octyle, nonyle ou décyle dont chacun est linéaire ou ramifié, un groupe halogénalkyle en $C_1$ à $C_6$, alcényle en $C_3$ à $C_6$, alcynyle en $C_3$ à $C_6$, (alkoxy en $C_1$ à $C_4$)-(alkyle en $C_1$ à $C_4$), (alkylthio en $C_1$ à $C_4$)-(alkyle en $C_1$ à $C_4$) ou un groupe cycloalkyle à noyau de 3 à 6 atomes, qui peut être interrompu par de l'oxygène, de l'azote ou du soufre, ou bien A est un groupe phényle, benzyle ou phénéthyle sans substituant ou portant chacun 1 à 5, notamment 1 à 3 substituants égaux ou différents, en choisissant dans chaque cas comme substituants : le fluor, le chlore, un radical méthyle, éthyle, n-propyle, isopropyle, méthoxy, éthoxy, trifluorométhyle, trifluorométhoxy, ou bien un radical phénoxy ou phénylthio sans substituant ou portant chacun 1 à 3 substituants fluoro, chloro, méthyle, méthoxy, trifluorométhyle et trifluorométhoxy égaux ou différents.

4. Procédé de production de dérivés de 4-alkoxy- et 4-(amino substitué)-3-aryl-pyrrolidones de formule générale (I)

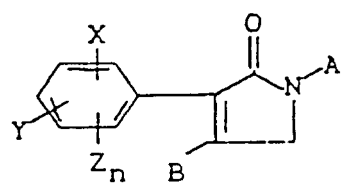

(I)

dans laquelle
X, Y, Z, n, A et B ont la définition indiquée dans la revendication 1,
caractérisé en ce que :

a) on obtient des composés de formule (I) dans lesquels B est un groupe hydroxyle et dans la formule desquels les restes A, X, Y et $Z_n$ ont la définition indiquée ci-dessus lorsque on effectue la condensation intramoléculaire d'ester de N-acylamino-acides de formule (II)

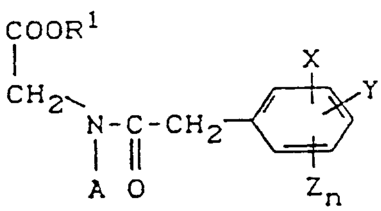

(II)

dans laquelle
A, X, Y, Z et n ont la définition indiquée ci-dessus, et

$R^1$ est un groupe alkyle, notamment méthyle ou éthyle,

en présence d'un diluant et en présence d'une base,
ou bien

b) on obtient des composés de formule (I) dans lesquels :

B est un groupe alkoxy, halogénalkoxy ou cycloalkoxy, le reste alkoxy étant non substitué ou substitué par un halogène et/ou par un radical alkyle, et

A, X, Y, Z et n ont la définition indiquée ci-dessus,
lorsqu'on fait réagir des composés de formule (I) dans lesquels B est un groupe hydroxyle avec des réactifs d'alkylation de formule (III)

$R^2 - X^1$ (III)

dans laquelle

$R^2$ est un groupe alkyle, halogénalkyle ou cycloalkyle, le reste cycloalkyle étant non substitué ou substitué par un halogène et/ou un radical alkyle, et

$X^1$ est un halogène ou un autre groupe partant classique pour des réactifs d'alkylation, le cas échéant en présence d'un diluant et en la présence éventuelle d'un accepteur d'acide,

ou bien

c) on obtient des composés de formule (I) dans lesquels B est un groupe

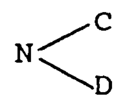

et

A, C, D, X, Y, Z et n ont la définition indiquée ci-dessus,

lorsqu'on fait réagir des composés de formule (I) dans lesquels B est un groupe hydroxyle, avec des amines de formule (IV)

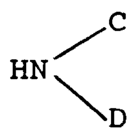

(IV)

dans laquelle

C et D ont la définition indiquée ci-dessus, le cas échéant en présence d'un diluant et en la présence éventuelle d'un agent déshydratant.

**5.** Compositions herbicides, caractérisées par une teneur en au moins un dérivé de 4-alkoxy- et 4-(amino substitué)-3-aryl-pyrrolidone de formule (I) suivant les revendications 1 à 4.

**6.** Procédé pour combattre des plantes non désirées, caractérisé en ce qu'on fait agir des dérivés de 4-alkoxy- ou 4-(amino substitué)-3-aryl-pyrrolidones de formule (I) suivant les revendications 1 à 4, sur les plantes et/ou sur leur milieu.

**7.** Utilisation de dérivés de 4-alkoxy- et 4-(amino substitué)-3-aryl-pyrrolidones de formule (I) suivant les revendications 1 à 4 pour combattre des plantes non désirées.

**8.** Procédé de préparation de compositions herbicides, caractérisé en ce qu'on mélange des dérivés de 4-alkoxy- et 4-(amino substitué)-3-aryl-pyrrolidones de formule (I) suivant les revendications 1 à 4 avec des diluants et/ou des substances tensio-actives.